(19) Europäisches Patentamt
European Patent Office
Office européen des brevets

(11) **EP 2 959 299 B1**

(12) **EUROPEAN PATENT SPECIFICATION**

(45) Date of publication and mention of the grant of the patent:
**02.01.2019 Bulletin 2019/01**

(51) Int Cl.:
*G01N 33/543* (2006.01)      *G01N 33/552* (2006.01)
*G01N 33/58* (2006.01)      *B32B 19/00* (2006.01)
*B05D 5/06* (2006.01)

(21) Application number: **14754877.0**

(22) Date of filing: **20.02.2014**

(86) International application number:
**PCT/US2014/017342**

(87) International publication number:
**WO 2014/130643 (28.08.2014 Gazette 2014/35)**

(54) **MULTILAYER FLUORESCENT NANOPARTICLES AND METHODS OF MAKING AND USING SAME**

MEHRSCHICHTIGE FLUORESZIERENDE NANOPARTIKEL UND VERFAHREN ZUR HERSTELLUNG DAVON UND VERWENDUNG DAVON

NANOPARTICULES FLUORESCENTES MULTICOUCHES ET LEURS PROCÉDÉS DE FABRICATION ET D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.02.2013 US 201361767066 P**

(43) Date of publication of application:
**30.12.2015 Bulletin 2015/53**

(73) Proprietor: **Cornell University**
**Ithaca, NY 14850 (US)**

(72) Inventors:
• **IYER, Srikant, K.**
**Atlanta, GA 30318 (US)**
• **WIESNER, Ulrich, B.**
**Ithaca, NY 14850 (US)**

(74) Representative: **Mathys & Squire LLP**
**The Shard**
**32 London Bridge Street**
**London SE1 9SG (GB)**

(56) References cited:
WO-A1-2011/042564      WO-A1-2011/042564
US-A1- 2009 297 448      US-A1- 2010 255 487
US-A1- 2011 204 258      US-A1- 2013 039 848

• WANG L ET AL: "Multicolor FRET silica nanoparticles by single wavelength excitation", NANO LETTERS, AMERICAN CHEMICAL SOCIETY, US, vol. 6, no. 1, 1 January 2006 (2006-01-01) , pages 84-88, XP002544357, ISSN: 1530-6984, DOI: 10-1021/NL052105B [retrieved on 2005-12-20]
• JINHYANG CHOI ET AL: "Core-shell silica nanoparticles as fluorescent labels for nanomedicine", JOURNAL OF BIOMEDICAL OPTICS, vol. 12, no. 6, 1 January 2007 (2007-01-01), page 064007, XP055133826, ISSN: 1083-3668, DOI: 10.1117/1.2823149
• ANDREW BURNS ET AL: "Core/Shell Fluorescent Silica Nanoparticles for Chemical Sensing: Towards Single-Particle Laboratories", SMALL, vol. 2, no. 6, 1 June 2006 (2006-06-01), pages 723-726, XP055133830, ISSN: 1613-6810, DOI: 10.1002/smll.200600017
• MIRIAM BENEZRA ET AL: "Multimodal silica nanoparticles are effective cancer-targeted probes in a model of human melanoma", JOURNAL OF CLINICAL INVESTIGATION, vol. 121, no. 7, 1 July 2011 (2011-07-01), pages 2768-2780, XP055295072, US ISSN: 0021-9738, DOI: 10.1172/JCI45600
• MA, KAI ET AL.: 'Ultrasmall sub-10 nm near-infrared fluorescent mesoporous silica nanoparticles.' JOURNAL OF THE AMERICAN CHEMICAL SOCIETY vol. 134.32, 2012, pages 13180 - 13183, XP055275745

## Description

This application claims priority to U.S. provisional patent application no. 61/767,066, filed February 20, 2013.

STATEMENT REGARDING FEDERALLY SPONSORED RESEARCH

This invention was made with government support under contract no. 2009-ST-108-LR0004 awarded by the Department of Homeland Security. The government has certain rights in the invention.

FIELD OF THE DISCLOSURE

The present disclosure generally relates to layered fluorescently responsive material-containing nanoparticles. More particularly, the present disclosure relates to multilayer, fluorescently responsive material-containing silica nanoparticles.

BACKGROUND OF THE DISCLOSURE

Drug discovery, drug screening, gene expression, and identification of proteins as vaccine targets are based on carrying out high throughput screening (HTS) assays involving large numbers of molecules. This requires screening large chemical libraries for particular target molecules such as proteins, antibodies, nucleotides or peptides. Screening large libraries or biological multiplexing accelerates the development of tools for therapeutic and diagnostic applications. Biological multiplexing involves conducting multiple assays simultaneously without compromising on sensitivity and specificity. Advancement towards miniaturized HTS has led towards screening over thousands of compounds a day. Top to bottom two dimensional array fabrication technology has led to development of DNA chips, microarrays and bioMEMS. Positional encoding based pattern recognition has further helped identifying specific molecular finger prints. Some of the major drawbacks with these technologies are that they are expensive in terms of equipment set-up and reagents and complex in terms of sample preparation and array fabrication. Furthermore, variability in measurements due to cross-reactivity and reproducibility has made these methodologies difficult for large scale analysis.

The potential of multiplex coding using color rationing based bead technology has previously been explored. Two organic dyes were used to encode a 5.5 $\mu$m polystyrene bead at 8 different intensity levels for multiplexed assays. 1.2 $\mu$m polystyrene beads encoded with six spectrally distinguishable CdSe/ZnS core-shell quantum dot nanoparticles at six different intensity levels in different ratios were synthesized by swelling the polymer in an appropriate solvent mixture. The fluorescent species in the above cases were all physically incorporated into micron sized polystyrene particles and because of their large size could not easily be applied for multiplexed *in-vivo* or intracellular bioimaging. Dendrimer like DNA (DL-DNA) based fluorescent nanobarcodes were developed by precisely tagging the DL-DNA with two organic fluorophores in different ratios based on the number of available reactive sites. In order to amplify the fluorescence signal from these nanobarcodes, again microbeads were used as support for imaging and molecular detection. Similarly, 70 nm Forster resonance energy transfer (FRET) based silica nanoparticles encoding dyes with different dye ratios were synthesized and loaded on 10 $\mu$m streptavidin coated microspheres. This work was extended and applied to optical encoding in combinatorial chemistry by developing > 100 nm (FRET) based silica particles encoding dyes with different dye ratios that were then loaded onto 10 $\mu$m polystyrene microspheres as substrates for multiplexing. The dye ratios encoded in these FRET based particles were quantified based on the dyes dosed into the reaction mixture and assuming all dyes were incorporated. Because of the large sizes of the final beads (> 1 $\mu$m) in none of these cases was intracellular imaging demonstrated.

Thus, there is a need to make multiplex coding particles where FRET is suppressed to obtain a desired level of particle brightness, and thus a desirable signal to noise, of sizes of about 100nm or less which can be used in *in vivo* and *in vitro* applications since particles with sizes below 100 nm are particularly suited for cellular uptake, and thus are desirable for intracellular imaging applications.

BRIEF SUMMARY OF THE DISCLOSURE

According to aspects of the invention, there are provided nanoparticles and methods according to the claims.

Disclosed herein are multicolor fluorescent silica nanoparticles (also referred to herein as multilayer, fluorescently responsive material (FRM)-containing nanoparticles, mcC dots, and tricolor C dots, and multicolor C dots) with surface PEG coatings (for functionalization with different moieties such as proteins, nucleic acids, small molecules). The multicolor fluorescent silica nanoparticles may be referred to as bright multicolor fluorescent silica nanoparticles. For example, the nanoparticles have sizes below 100 nm that contain two, three or more spectrally distinct dyes at two, three or more different intensity levels. The multicolor fluorescent silica nanoparticles can be used, for example, for intracellular bioimaging, high throughput screening and medical diagnostics.

In an instance, the silica nanoparticles contain the dyes N-(7-dimethylamino-4-methylcoumarin-3-yl) maleimide (DACm (blue)), tetramethylrhodamine-5-maleimide (TMRm (green)) and Cy5-maleimide (Cy5m (red)). These dyes are contained at three intensity levels per dye (no dye, low dye, or high dye). The particle architecture is designed so to control the number of dyes per color and to minimize energy transfer between dyes for maximum brightness. This combination of three dyes at three intensity levels results in the synthesis of twenty-six distinguishable particles based on wavelength and fluorescence intensity. The nanoparticles have one to three orders of magnitude in fluorescence brightness enhancement compared to free dyes. Nanoparticles with high dye loading were ~ 3.5-4 times brighter than particles with medium dye loaded particles. In this system moving from medium to high dye loadings by incorporating additional silica shells does not decrease the relative fluorescence emission of the nanoparticles.

The methods of making multicolor fluorescent silica nanoparticles are carried out such that, e.g., dyes are added to a dye doped particle core in a layer-by-layer fashion and each spectrally distinct dye is spatially separated by a pure silica shell in order to reduce energy transfer between the dyes.

In an instance, the nanoparticles are synthesized such that the three dyes are added in a layer-by-layer fashion with green in the core followed by red in an inner shell followed by blue added as the final dye layer. However any order of dye addition can be performed without impacting the performance of the nanoparticle. Dye layers are spatially separated by thick enough silica shells to effectively suppress energy transfer between dye layers. Thus each individual particle has onion-type structures with, for example, up to twenty-four distinct layers around a dyed core.

BRIEF DESCRIPTION OF THE FIGURES

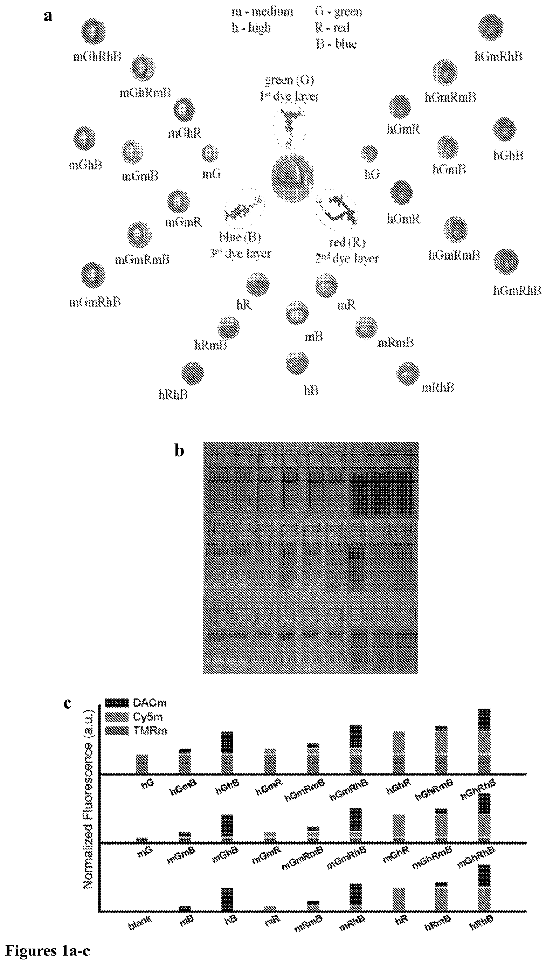

Figure 1. (a) Schematic illustration of layer-by-layer approach for incorporating the three dyes TMRm (green), Cy5m (red) and DACm (blue) in silica nanoparticles (center) and the possible combinations based on the encapsulation levels for each dye (0, 5, 20 dyes, respectively). For simplicity, particles with medium dye loading (~5 dyes/particle, lighter shade) and high dye loading (~20 dyes/particle, darker shade) are shown with a lighter/darker shade of green, red and blue. (b) Photo taken of the 26 synthesized core-shell multicolor fluorescent nanoparticles in water arranged in different rows as particles with no TMRm (no green, bottom row), particles with ~5 TMRm (low green dye loading, middle row) and particles with ~20 TMRm dyes in the core (high green dye loading, top row). Note that the 27th cuvette on the lowest row (left) depicts a solution of pure silica particles without any dye, i.e. with color combination 0, 0, 0. (c) Key to the nanoparticles shown in Figure 1b. Blank represents pure silica particles without any dye, as a control. TMRm (green), Cy5m (red) and DACm (blue)

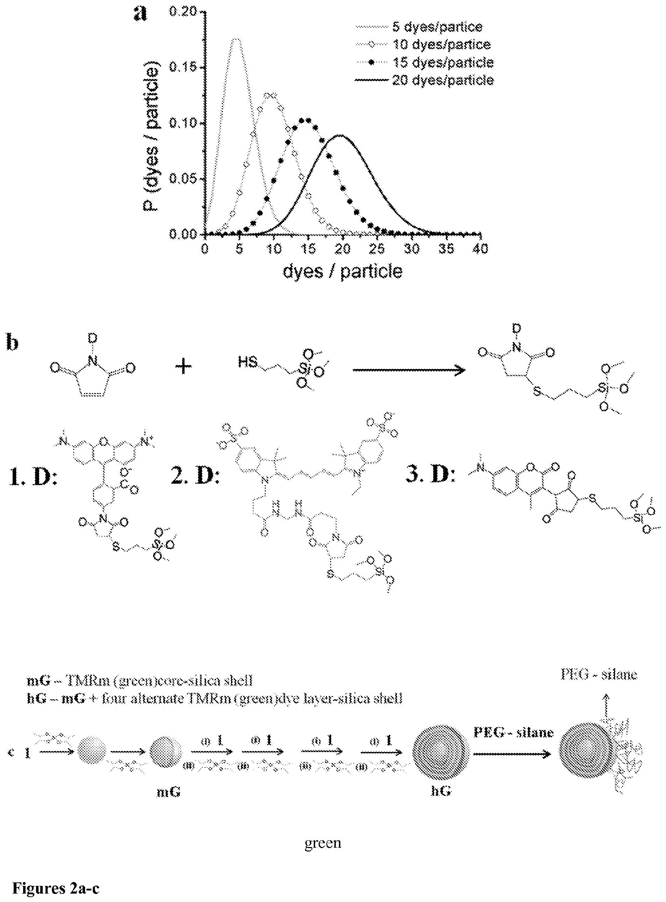

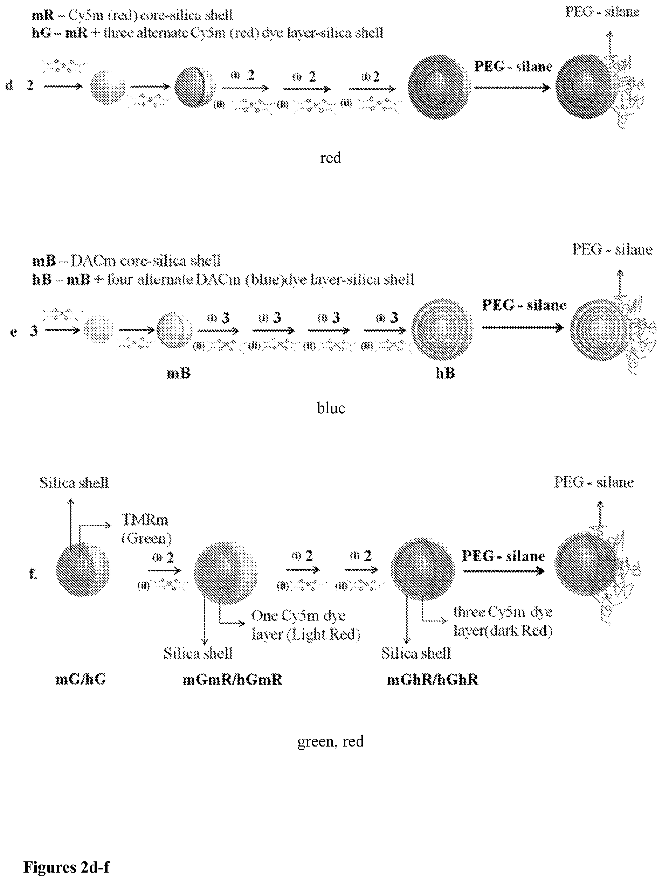

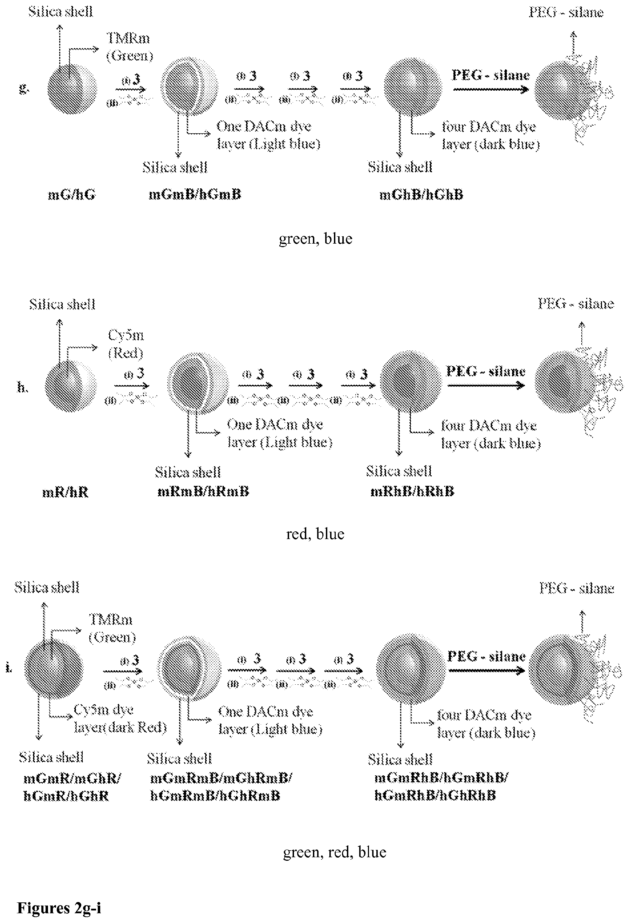

Figure 2. Schematic representations of *multicolor* C dots (*mc*C dots) synthesis: (a) Plot comparing the overlap between Poisson distributions for synthesis batches with different average numbers of dyes per particle, see legend. Order of curves from left to right: 5, 10, 15, 20 dyes/particle. (b) Generalized reaction schematic of maleimide derivative of the three dyes TMRm (1), Cy5m (2) and DACm (3) with (3-mercaptopropyl)-trimethoxysilane to give the respective dye-silane conjugate. (c-e) Layer-by-layer dye addition for single color C dots: TMRm particles (G), Cy5m particles (R) and DACm particles (B), followed by the addition of a 5k molar mass polyethylene glycol silane (PEG-silane) as the final layer; (f-h) layer-by-layer approach to dual color *mc*C dots with different brightness levels for (f) G and R, (g) G and B and (h) R and B combinations starting from G and R single color C dots, followed by capping with PEG-silane; and (i) layer-by-layer approach to triple color C dots capped with PEG-silane starting from G and R containing dual color C dots.

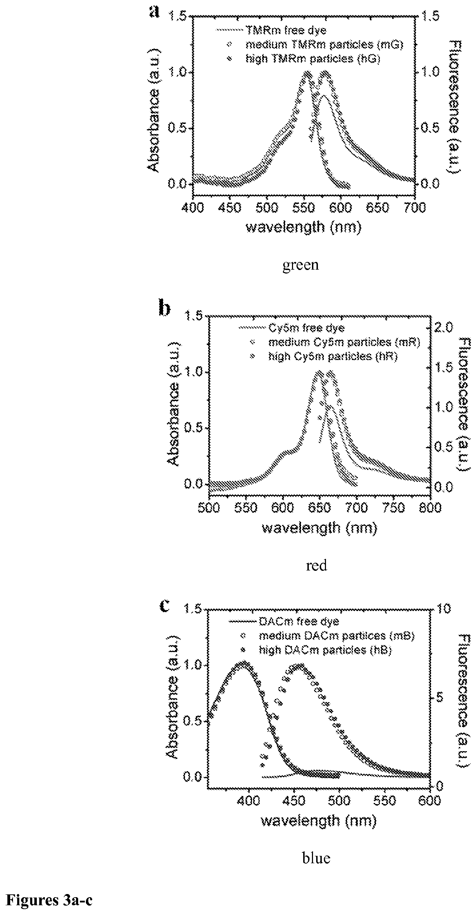

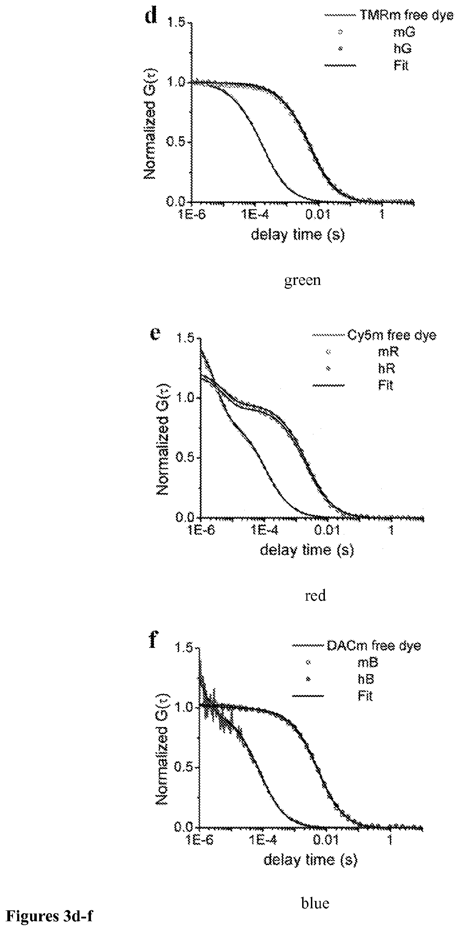

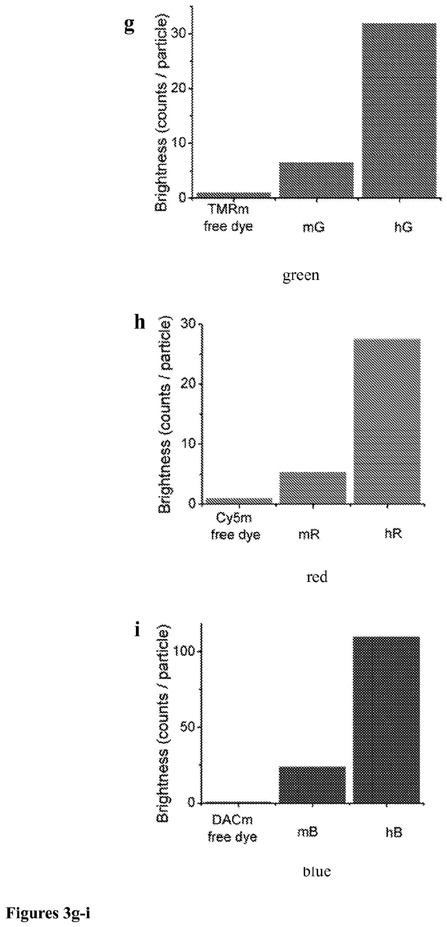

Figure 3. Characterization of single color C dots. Panels (a-c) compare the absorbance and fluorescence spectra of medium and high dye loaded nanoparticles to the parent free dye for (a) TMRm, (b) Cy5m and (c) DACm. Solutions of free dye and particles were absorbance matched before these measurements. Panels (d-f) compare the FCS autocorrelation curves of the free dyes (solid lines) with those of medium (hollow circles) and high (solid circles) dye loaded nanoparticles in solutions: (d) TMRm, (e) Cy5m and (f) DACm. Panels (g-i) compare the brightness per fluorescent species (dyes vs particles) as measured from FCS detector data sets for (g) TMRm system, (h) Cy5m system and (i) DACm system.

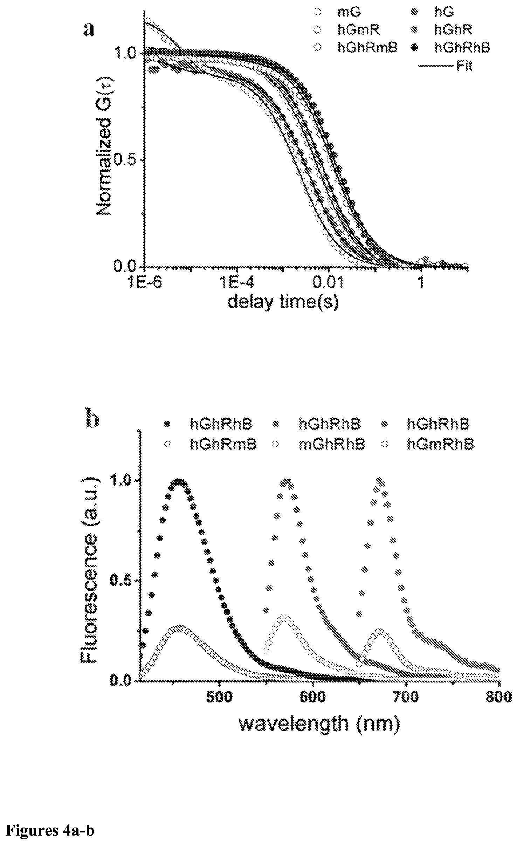

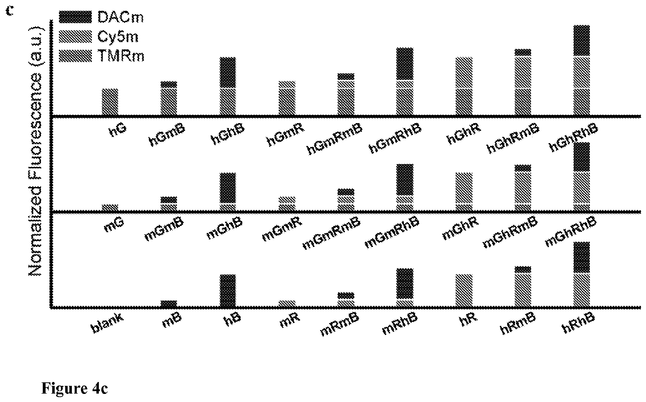

Figure 4. Characterization *of mc*C dots. (a) Representative FCS curves of specific particle intermediates in solution moving towards triple color C dots covering the entire synthetic scheme starting from medium/high TMRm dye loaded single color C dots (mG, hollow green circles/hG, solid green circles) via medium/high Cy5m dye loaded dual-color C dots (hGmR, hollow red circles/hGhR, solid red circles) to the final triple color C dots with medium/high DACm dye loadings (hGhRmB, hollow blue circles/hGhRhB, solid blue circles). Blue (left curve), green (middle curve), red (right curve). (b) Representative fluorescence emission spectra of triple color C dots as obtained by excitation in the blue (left), green (middle) and red (right). (c) Summary of experimental intensity profiles for each of the 26 fluorescent silica nanoparticles as measured by steady-state fluorescence emission spectra. Green bars represent emission from TMRm, red bars from Cy5m and blue bars from DACm. Bar heights represent emission intensity levels.

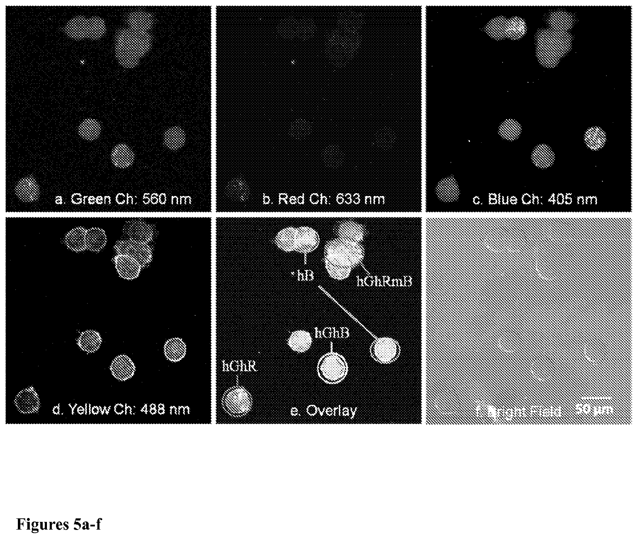

Figure 5. Confocal fluorescence microscopy images *of mc*C dots in RBL-2H3 mast cells showing channel, (a) Green Ch: 560 nm, (b) Red Ch: 633 nm, (c) Blue Ch: 405 nm, (d) Yellow Ch: 488 nm; (e) overlaid images of (a, b, c, and d) (f) bright-field image.

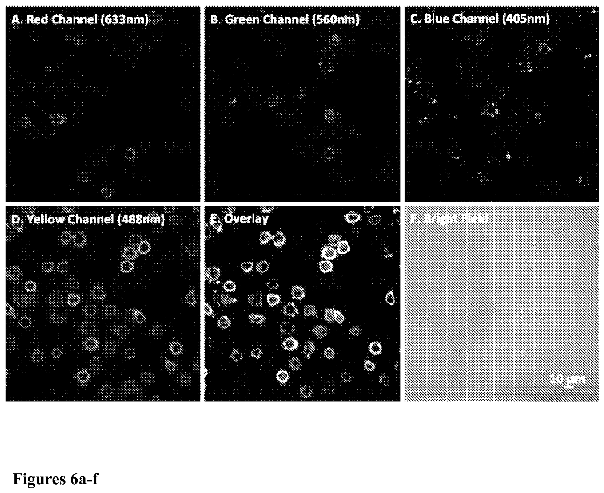

Figure 6. A single 512 x 512 mixed-particle image tile showing cells that contain up to 17 different particles. (a-d) Images were acquired simultaneously in the red, green, blue, and yellow channels. (e) All four fluorescent images were stacked to demonstrate that particles are in fact inside the cells. (f) Image acquired in bright field.

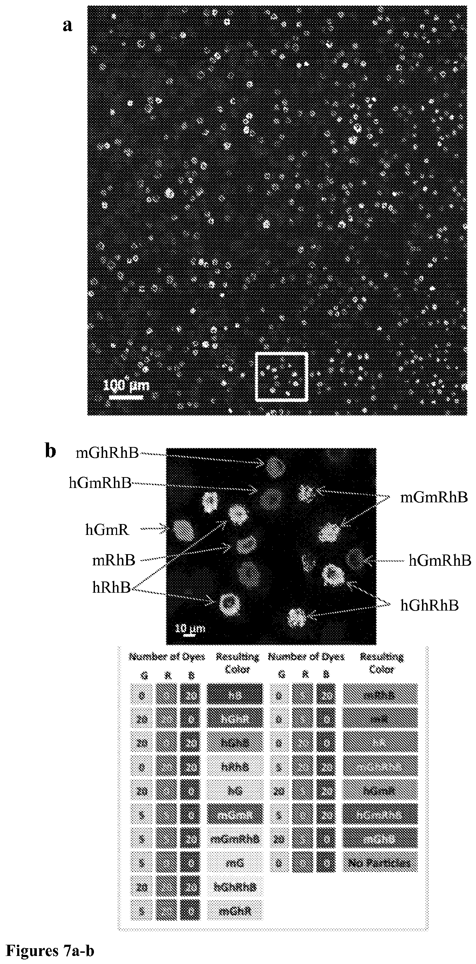

Figure 7. 17 spectrally distinct multicolor nanoparticles were loaded into Rat Basophilic Leukemia cells. (a) Full size (1280 x 1280 pixel) false color image of cells each loaded with 1 of 17 single "color" *mc* C Dots demonstrating successful "decoding" of a multi-particle sample. A color-coded legend, illustrating both particle composition and assigned cell color, is shown at the bottom right. (b) A magnified region of the same image.

## DETAILED DESCRIPTION OF THE DISCLOSURE

The present disclosure provides multilayer, fluorescently responsive material (FRM)-containing nanoparticles. Also provided are methods of making and using the multilayer, FRM-containing nanoparticles.

A multilayer, FRM-containing nanoparticle of the present disclosure provides a nanoparticle that can be uniquely identified by the specific FRM(s) and FRM level(s). The nanoparticles with built-in codes for identification can act as carriers and labels for molecules. The specific FHM(s)/level(s) combination (that may be described as an optical "bar code") can be identified by its fluorescence emission (e.g., in the form of an image). For example, in a mixture of multilayer, FRM-containing nanoparticles having different FHM/level combinations each different FHM/level combination can be uniquely identified. Each distinguishable particle is analogous to a single well or array in the 2-D array technology. Hence multiplexing is not restricted to the number of wells or arrays, but the number of distinguishable particles. The nanoparticle architecture minimizes intralayer and interlayer quenching of the fluorescence from the FRM. This provides nanoparticles having a desirable brightness via effective use of all fluorescent dyes in the particles, resulting in desirable signal-to-noise levels in optical/fluorescence detection schemes.

In an aspect, the present disclosure provides multilayer, FRM-containing silica nanoparticles. The nanoparticles comprise one or more fluorescently responsive materials (FRMs) which can be present at one or more amounts (i.e., levels). The nanoparticles can be from 5 nm to 500 nm, including all integer values and ranges therebetween, in size (e.g., diameter).

Reference to multilayer, FRM-containing nanoparticle(s) herein is intended to include multilayer, dye-containing nanoparticle(s). Reference to FRM-free silica layer(s) herein is intended to include dye-free silica layer(s). Reference to FRM-containing silica layer(s) herein is intended to include dye-containing silica layer(s). Reference to outermost FRM-free silica layer(s) herein is intended to include outermost dye-containing silica layer(s). Reference to FRM herein is intended to include dye molecule(s). Reference to FRM-conjugate precursor(s) herein is intended to include dye-conjugate precursor(s). Reference to outermost FRM-containing silica layer(s) herein is intended to include outermost dye-containing silica layer(s).

In an embodiment, the nanoparticle comprises a silica core comprising a plurality of FRM molecules covalently bound to the silica network of the core, 1 to 100 FRM-containing silica layers, each layer comprising a plurality of FRM molecules covalently bound to the silica network of the FRM-containing silica layer, one or more FRM-free silica layers, wherein one of the FRM-free silica layers separates the silica core from one of the FRM-containing silica layers and, if present, each adjacent pair of the FRM-containing silica layers is separated by one of the FRM-free silica layers, an outermost FRM-free silica layer disposed on the outermost FRM-containing silica layer; and a plurality of poly(ethylene glycol) molecules covalently bound to the outer surface of the outermost FRM-free silica layer. The nanoparticle may further comprise one or more moieties (e.g., proteins, peptides, nucleic acids, aptamers, antibodies, antibody fragments, polymers, organic small molecules, and combinations thereof) covalently bound to the poly(ethylene glycol) molecules that are covalently bound to the outer surface of the outermost FRM-free silica layer. The nanoparticle can have a diameter of 5 nm to 500 nm, including all integer nm values and ranges therebetween. Each FRM-free silica layer has a thickness (e.g., 1 nm to 20 nm) such that there is less than 10% measurable energy transfer between the FRM in the core and in an adjacent FRM-containing silica layer or in adjacent FRM-containing silica layers.

In another embodiment, the nanoparticle comprises a silica core comprising a plurality of FRM molecules covalently bound to the silica network of the core, 1 to 100 FRM-containing silica layers, each layer comprising a plurality of FRM molecules covalently bound to the silica network of the FRM-containing silica layer, one or more FRM-free silica layers, wherein one of the FRM-free silica layers separates the silica core from one of the FRM-containing silica layers and, if present, each adjacent pair of the FRM-containing silica layers is separated by one of the FRM-free silica layers, and an outermost FRM-free silica layer disposed on the outermost FRM-containing silica layer. The nanoparticle can have a diameter of 5 nm to 500 nm, including all integer nm values and ranges therebetween. Each FRM-free silica layer has a thickness (e.g., 1 nm to 20 nm) such that there is less than 10% measurable energy transfer between the FRM in the core and in an adjacent FRM-containing silica layer or in adjacent FRM-containing silica layers.

In an embodiment, each of the FRM-free silica layers may be disposed on the silica core or an FRM-containing silica layer.

In another embodiment, the nanoparticle comprises a) a silica core comprising a plurality of FRM covalently bound to the silica network of the core; and b) alternating layers of FRM-free silica layers and FRM-containing layers surrounding the silica core, wherein a FRM-free silica layer surrounds the core. The outermost layer is an FRM-free silica layer or an FRM-containing layer. Optionally, a plurality of polyether molecules are covalently bound the outer surface of the outermost FRM-free silica layer or FRM-containing layer.

The core is substantially spherical in shape and can be from 1 nm to 20 nm, including all integer nm values therebetween, in size (e.g., diameter). The core has a plurality of fluorescently responsive material (e.g., fluorescently responsive molecules such as dye molecules and pigment molecules) that can be covalently bound to the silicon oxide network of the core.

The FRM-free silica layer is disposed on the core and/or a FRM-containing silica layer. There is no detectible FRM in this layer. This layer at least partially covers the core and/or a FRM-containing silica layer. In an embodiment, the layer is a continuous layer that completely covers the core and/or a FRM-containing silica layer. The nanoparticle can have from 1 to 99 FRM-free silica layers, including all integer numbers of layers and ranges therebetween.

Each FRM-free silica layer isolates (i.e., reduces energy transfer between) the core and adjacent FRM-containing silica layer or adjacent FRM-containing silica layers. In various embodiments, each FRM-free silica layer has a thickness such that there is 10% or less, 5% or less, 4% or less, 3% or less, 2% or less, or 1% or less measurable energy transfer between the FRM molecules in the core and in an adjacent FRM-containing silica layer or in adjacent FRM-containing silica layers. In an embodiment, each FRM-free silica layer has a thickness such that there is no measurable energy transfer between the FRM in the core and adjacent FRM-containing silica layer or adjacent FRM-containing silica layers. Energy transfer between the FRM in the core and adjacent FRM-containing silica layer or adjacent FRM-containing silica layers can be measured by methods known in the art. For example, energy transfer can be measured by Energy transfer can be measured by excitation of one FRM (e.g., one dye species) (donor) absorbing at lower wavelengths and examination of the emission of a second FRM (e.g., a second dye species) (acceptor) that has an overlap between its absorbance spectrum and the emission spectrum of the donor dye. A second method is to measure the emission intensity of the donor FRM (e.g., dye) in absence and presence of an acceptor FRM (e.g., dye). If FRET takes place between donor and acceptor, then the donor emission is suppressed. For example, the FRM-free silica layer can be from 1 nm to 20 nm, including all integer nm values and ranges therebetween.

The FRM-containing silica layer is disposed on a FRM-free silica layer. This layer at least partially covers the FRM-free silica layer. In an embodiment, the layer is a continuous layer that completely covers the core and/or a FRM-containing silica layer. In an embodiment, each FRM-free silica layer has a single type of FRM. The nanoparticle can have 1 to 100 FRM-containing silica layers, include all integer numbers of layers and ranges therebetween. The FRM-containing silica layer can have a thickness of less than 1 nm to 20 nm. In an embodiment, the FRM-containing silica layer has a thickness of 1 nm to 20 nm, including all integer nm values and ranges therebetween. The outermost FRM-free silica layer is disposed on the outermost FRM-containing silica layer. This layer at least partially covers the outermost FRM-containing silica layer. In an embodiment, the layer is a continuous layer that completely covers the core and/or a FRM-containing silica layer. This layer has a thickness of 1 nm to 20 nm, including all integer nm values and ranges therebetween.

The core, FRM-containing silica layers, and FRM-free silica layers have a network structure. The network can be formed by condensation of a silica precursor. In an embodiment, the network is a silica network. The silica network of the core can be formed by condensation of silica precursors such as, for example, TMOS and TEOS. Suitable precursors can be made using known methods and can be obtained from commercial sources.

Any fluorescently responsive material (e.g., dyes and pigments) that can be covalently bound to the silicon oxide network of the nanoparticle can be used. Examples of FRMs include dye molecules. Suitable fluorescently responsive materials can be made using known methods and can be obtained from commercial sources.

A wide variety of suitable chemically reactive fluorescently responsive materials are known, see for example MOLECULAR PROBES HANDBOOK OF FLUORESCENT PROBES AND RESEARCH CHEMICALS, 6th ed., R. P. Haugland, ed. (1996). A typical fluorophore is, for example, a fluorescent aromatic or heteroaromatic compound such as is a pyrene, an anthracene, a naphthalene, an acridine, a stilbene, an indole or benzindole, an oxazole or benzoxazole, a thiazole or benzothiazole, a 4-amino-7-nitrobenz-2-oxa-1,3-diazole (NBD), a cyanine, a carbocyanine, a carbostyryl, a porphyrin, a salicylate, an anthranilate, an azulene, a perylene, a pyridine, a quinoline, a coumarin (including hydroxycoumarins and aminocoumarins and fluorinated derivatives thereof), and like compounds, see for example U.S. Pat. Nos. 5,830,912; 4,774,339; 5,187,288; 5,248,782; 5,274,113; 5,433,896; 4,810,636; and 4,812,409.

For example, the dye is an organic dye. Examples of suitable organic dyes include, but are not xanthene derivatives (e.g., fluorescein, rhodamine, Oregon green, eosin, Texas red, and Cal Fluor dyes), cyanine derivatives (e.g.,

cyanine, indocarbocyanine, oxacarbocyanine, thiacarbocyanine, merocyanine, and Quasar dyes), naphthalene derivatives (e.g., dansyl and prodan derivatives), coumarin derivatives, oxadiazole derivatives (e.g., pyridyloxazole, nitrobenzoxadiazole and benzoxadiazole), pyrene derivatives (e.g., cascade blue), oxazine derivatives (e.g., Nile red, Nile blue, cresyl violet, oxazine 170), acridine derivatives (e.g., proflavin, acridine orange, acridine yellow), arylmethine derivatives (e.g., auramine, crystal violet, malachite green), tetrapyrrole derivatives (e.g., porphin, phtalocyanine and bilirubin), CF™ dye (Biotium), BODIPY® (Invitrogen), ALEXA FLUOR® (Invitrogen), DYLIGHT™ (Thermo Scientific, Pierce), ATTO™ and TRACY™ (Sigma Aldrich), FLUOPROBES® (Interchim), derivatives thereof, and the like.

It is desirable to use fluorescently responsive materials that have absorption and/or emission spectra that correspond to commonly used excitation and/detection wavelengths. For example, the fluorescently responsive materials that have absorption and/or emission spectra that correspond to excitation and/detection wavelengths used in commercially available imaging systems. For example, the nanoparticle can have FRM with red, green, blue emission wavelengths, or a combination thereof. In an embodiment, the fluorescently responsive materials are fluorescent dyes selected from N-(7-dimethylamino-4-methylcoumarin-3-yl) (DAC), tetramethylrhodamine-5-maleimide (TMR), Cy5, N-(7-dimethylamino-4-methylcoumarin-3 -yl)maleimide (DACm), tetramethylrhodamine-5 - maleimide (TMRm), Cy5-maleimide (Cy5m), or a combination thereof.

The FRM is covalently bound (e.g., directly or via a linking group) to the nanoparticle silica network of the core or FRM-containing layer. The FRM can be covalently bound directly to the silica network via a linking group through, for example, an ester bond, amide bond, or thioether bond. For example, a FRM (e.g., dye molecule) can be covalently bound to a sol-gel precursor (e.g., a functionalized alkyl(trialkoxy)silane) though a linking group and this FRM-conjugate precursor is used to produce the core or FRM-containing silica layer. In an embodiment, the FRM is covalently bound to the silicon oxide network by a maleimide-mercapto conjugate. Covalent bond or bonds between the FRM and a sol-gel precursor (e.g., a functionalized alkyl(trialkoxy)silane) can be formed by methods known in the art.

The FRM (e.g., dye molecules) in the nanoparticles (i.e., covalently bound to the nanoparticle silica network of the core or FRM-containing layer) have fluorescence emission intensity (i.e., brightness) that is greater than that of the free FRM when the fluorescence emission of each is measured in an aqueous solution. In various embodiments, the fluorescence emission intensity of one or more or all the FRM (e.g., dye molecules) in the nanoparticle is 10%, 20%, 30%, 40%, 50%, 75%, 100%, two times, five times, 10 times, or 20 times greater than that of the free-FRM (e.g. dye molecules) when the fluorescence emission of each is measured in an aqueous solution.

The outermost FRM-free silica layer may be passivated such that the nanoparticle is sterically stabilized against aggregation with other nanoparticles in physiological buffer solutions. For example, the nanoparticle has a plurality of polymer groups, such as polyether groups covalently bound to the outer surface of the layer. Polyether groups can be derived from a polyether molecule or functionalized polyether molecule. In an embodiment, the polyether group is a PEG group.

In an embodiment, the nanoparticle has a plurality of poly(ethylene glycol) (PEG) groups covalently bound to the outer surface of the layer. The PEG groups can be derived from a PEG molecule or functionalized PEG molecule. The PEG groups are covalently bound to the silica network of the layer. For example, the PEG groups can have molecular weight of 400 g/mol to 10,000 g/mol. The PEG groups cover at least a portion of the outermost FRM-free silica layer. It is desirable that the PEG groups cover at least a portion of the layer such that the nanoparticles are sterically stabilized against aggregation in physiological buffer solutions.

One or more of the PEG groups may have a moiety covalently bound to the group (i.e., PEG chain). In an embodiment, all of the PEG groups have a moiety covalently bound to the PEG group (i.e., PEG chain). The nanoparticle can have all the same moiety or combinations of moieties. The moiety can be a targeting moiety that targets a cellular structure (e.g., a surface cellular structure or intracellular structure). Examples of suitable moieties include, but are not limited to, proteins (e.g., protein subunits and protein domains), peptides, nucleic acids (e.g., single-stranded DNA molecules, double-stranded DNA molecules, single-stranded RNA molecules, double-stranded RNA molecules, and branched DNA molecules), aptamers (e.g,. DNA aptamers, RNA aptamers, and protein aptamers), antibodies, antibody fragments, polymers (e.g., dendrimers), organic small molecules (e.g., drug molecules and organic compounds such as folate or folic acid).

The FRM (e.g., individual dyes) can be present in the nanoparticle at various amounts (i.e., levels). The FRM present in the nanoparticle is the sum of all the same type of FRM present in any of the core and FRM-containing silica layers. The amount (i.e., level) of FRM in a given nanoparticle can be expressed as the number of FRM (e.g., dye molecules) per nanoparticle. It is desirable that the amount of dye is such that the levels of the FRM in a nanoparticle can be distinguished from the levels of the same FRM in a different nanoparticle. The absence of the dye is considered a level. The amount of dye present in nanoparticles having the same nominal level of dye may vary. In various embodiments, the amount of FRM in the nanoparticle is such that there is 10% or less, 5% or less, 4% or less, 3% or less, 2% or less, 1% or less overlap in the amount of the same FRM in another nanoparticle present (e.g., constituting another optical barcode). In an embodiment, the amount of FRM in the nanoparticle is such that there is no overlap in the amount of the same FRM in another nanoparticle present (e.g., constituting another optical barcode).

In another aspect, the present disclosure provides a composition comprising the nanoparticles. The composition can comprise a plurality of distinguishable (i.e., uniquely identifiable) nanoparticles. In an embodiment, the composition comprises a plurality of nanoparticles. In another embodiment, composition comprises a plurality of nanoparticles having different combinations of dyes and/or levels of dyes. For example, each of the plurality of nanoparticles has two or three dyes (e.g., N-(7-dimethylamino-4-methylcoumarin-3-yl) (DAC), tetramethylrhodamine-5-maleimide (TMR), Cy5) at a level of 0, 5, or 20 dyes per nanoparticle.

In another aspect, the present disclosure provides methods of making the multilayer, FRM-containing nanoparticles. In the methods, the nanoparticle is formed by layer-by-layer addition of FRM-free silica layers and FRM-containing silica layers to a core.

In an embodiment, a method of making a multilayer, FRM-containing nanoparticle comprises the steps of: a) contacting a silica precursor, a plurality of a single type of FRM conjugate precursor, a solvent, and base such that a silica core having a plurality of FRM is conjugated to the silica network of the silica core are formed, b) contacting the material from step a) with a silica precursor and a solvent such that a FRM-free silica layer is formed on the silica core, c) contacting the material from b) with a silica precursor, a single type of FRM conjugate precursor, a solvent, and base such that a FRM-containing silica layer is formed, contacting the material from b) with a silica precursor, a single type of FRM conjugate precursor, a solvent, and base such that a FRM-containing silica layer is formed; d) optionally, contacting the material from step c) with a silica precursor and a solvent such that a FRM-free silica layer is formed on the FRM-containing silica layer is formed and contacting the resulting material with a silica precursor, a single type of FRM conjugate precursor, a solvent, and base such that a FRM-containing silica layer is formed; e) optionally, repeating step d) a desired number of time, wherein the contacting is to the material from a previously carried out step d); e) contacting the material from step c), step d), or step e) with a silica precursor and a solvent such that an outermost FRM-free silica layer is formed on the outermost FRM-containing silica layer; f) contacting the material from step c), d, or step e) with a silica precursor and a solvent such that an outermost FRM-free silica layer is formed on the outermost FRM-containing layer; and g) contacting the material from step f) with functionalized polyether molecules such that a nanoparticle having a plurality of polyether molecules covalently bound to the outer surface of the outermost FRM-free silica layer of the nanoparticle is formed. In an example, the functionalized polyether molecules are functionalized PEG molecules and the polyether groups are PEG groups.

In another embodiment, a method of making a multilayer, FRM-containing nanoparticle comprises the steps of: a) contacting a silica precursor, a plurality of a single type of FRM conjugate precursor, a solvent, and base such that a silica core having a plurality of FRM is conjugated to the silica network of the silica core are formed, b) contacting the material from step a) with a silica precursor and a solvent such that a FRM-free silica layer is formed on the silica core, c) contacting the material from b) with a silica precursor, a single type of FRM conjugate precursor, a solvent, and base such that a FRM-containing silica layer is formed, contacting the material from b) with a silica precursor, a single type of FRM conjugate precursor, a solvent, and base such that a FRM-containing silica layer is formed; d) optionally, contacting the material from step c) with a silica precursor and a solvent such that a FRM-free silica layer is formed on the FRM-containing silica layer is formed and contacting the resulting material with a silica precursor, a single type of FRM conjugate precursor, a solvent, and base such that a FRM-containing silica layer is formed; e) optionally, repeating step d) a desired number of time, wherein the contacting is to the material from a previously carried out step d); f) contacting the material from step c), step d), or step e) with a silica precursor and a solvent such that an outermost FRM-free silica layer is formed on the outermost FRM-containing silica layer.

In the embodiments making a multilayer, FRM-containing nanoparticle, the solvents, bases, FRM conjugate precursors, and silica precursors can be any species disclosed herein. Thus, the solvents, bases, FRM conjugate precursors, and silica precursors can be the same for each contacting step or may be different for one or more of the contacting steps.

The silica precursor undergoes a reaction (such as a condensation reaction) to form a silica network. The silica precursor can tetramethoxysilane (TMOS) or tetraethoxysilane (TEOS). The precursors can be made using methods known in the art or obtained from commercial sources.

The FRM conjugate precursor is a FRM covalently bound, directly or via a linking group, to a FRM conjugate moiety that has at least one group (e.g., alkoxysilane group such as methoxysilane or ethoxysilane) that can undergo a condensation reaction such that the FRM is covalently bound to the network (e.g., silica network) of the core or FRM-containing silica layer. For example, the FRM conjugate moiety is a trialkoxysilane group (e.g., $-Si(OR)_3$, where each R each is a methyl or ethyl group) bearing a reactive group such as a mercapto group (-S-H). The linking group can be derived from a maleimide group. The FRM conjugate precursor can be formed by known conjugation chemistries. In an embodiment, FRM conjugate precursor is a maleimide-mercapto conjugate. For example, the FRM is conjugated to 3-mercaptopropyl-trimethoxysilane (MPTMS) directly or via reaction with a maleimide group on a FRM-maleimide conjugate. In an embodiment, the FRM is a FRM-maleimide conjugate. For example, a FRM-maleimide conjugate is reacted with 3-mercaptopropyl-trimethoxysilane (MPTMS) to form a FRM conjugate precursor.

The functionalized polyether molecule (e.g., functionalized PEG molecule) has a functional group that can react with the outermost surface of the outermost FRM-free silica layer such that the polyether molecule (e.g., PEG molecule) is covalently bound to the outer surface of the outermost FRM-free silica layer of the nanoparticle. For example, a polyether molecule or PEG molecule is covalently bound, directly or via a linking group, to a moiety that has at least one group (e.g., alkoxysilane group such as methoxysilane or ethoxysilane) that can undergo a condensation reaction such that the polyether molecule or PEG molecule is covalently bound to the silica network of the outermost surface of the outermost FRM-free silica layer. An example of a suitable functional group is a trialkoxysilane group (e.g., $-Si(OR)_3$, where each R is a methyl or ethyl group). An example of a suitable linker group is ($-O-CH_2-CH_2CH_2-$). In an embodiment, the functionalized PEG molecule is methoxy-PEG-silane (mPEG-silane).

Optionally, the functionalized PEG molecule can have a moiety covalently bound to it. Such a functionalized PEG molecule can be formed using a heterobifunctional PEG molecule. The heterobifunctional PEG molecule has at least one functional group that can react with a functional group on the outermost surface of the outermost FRM-free silica layer and a functional group that can react with the moiety (or a functionalized version thereof).

In the core forming reaction, the silica precursor, a plurality of a single type of FRM conjugate precursor, a solvent, and base (e.g., ammonia) are reacted under conditions (e.g., time and temperature) such that a plurality of silica cores having a plurality of FRM conjugated to the silica network of the silica core are formed.

In the FRM-free silica layer forming reaction, the cores (e.g., the reaction mixture in which the cores are formed) are contacted with a silica precursor and a solvent such that a FRM-free silica layer is formed on the silica core.

In the FRM-containing silica layer forming reaction, the nanoparticles comprising a core and FRM-free silica layer (e.g., the reaction mixture in which the nanoparticles comprising a core and FRM-free silica layer are formed) are contacted with a silica precursor, a single type of FRM conjugate precursor different than the single type of FRM conjugate precursor in the core, a solvent, and base such that a FRM-containing silica layer is formed.

In the FRM-containing silica layer forming reaction the concentration of silica precursor is kept below the nucleation threshold concentration. In one embodiment, the reaction is added in serial aliquots to keep the concentration of silica precursor below the nucleation threshold concentration. It is desirable to use TMOS, which has faster hydrolysis reaction kinetics as compared with those of TEOS, in order to make it easier to keep the concentration of silica precursor below the nucleation threshold concentration.

Optionally, the FRM-free silica layer forming reaction and FRM-containing silica layer forming reaction are repeated sequentially on the nanoparticles comprising in sequence from the center of the nanoparticle a core, FRM-free silica layer, and FRM-containing silica layer (e.g., the reaction mixture in which these nanoparticles are formed) until nanoparticles having 2 to 100 FRM-containing silica layers, including all integer number of FRM-containing silica layers and ranges there between, are formed. In these optional FRM-containing silica layer reactions, the FRM can be the same or different than that used in any other FRM-containing silica layer reactions

In the outermost FRM-free silica layer forming reaction, the nanoparticles having a core, one or more FRM-free silica layers, and one or more FRM-containing silica layers (e.g., the reaction mixture in which these nanoparticles are formed) are contacted with a silica precursor and a solvent such that an outermost FRM-free silica layer is formed on the outermost FRM-containing silica layer.

In the PEG functionalizing reaction (also referred to herein as PEGylation), the nanoparticles having a core, one or more FRM-free silica layers, and one or more FRM-containing silica layers, and an outermost FRM-free silica layer (e.g., the reaction mixture in which these nanoparticles are formed) are contacted with functionalized PEG molecules such that a nanoparticle having a plurality of PEG molecules covalently bound to the outer surface of the outermost FRM-free silica layer of the nanoparticle are formed.

In an embodiment, a silica core is formed using a single type of FRM conjugate precursor, a solvent, and a base. An FRM-free silica layer is formed on the silica core using a silica precursor and a solvent. An FRM-containing silica layer is formed on the FRM-free silica layer using a silica precursor, a single type of FRM conjugate precursor, a solvent, and a base. Optionally, one or more FRM-free silica layers and one or more FRM-containing silica layers are formed in an alternating manner on the FRM-containing silica layer farthest from the silica core. After all FRM-containing layers are formed, an outermost FRM-free silica layer is formed on the FRM-containing layer that is farthest from the silica core using a silica precursor and a solvent. The outermost FRM-free silica layer is optionally reacted with functionalized polyether molecules such that a nanoparticle having a plurality of polyether molecules covalently bound to the outer surface of the outermost FRM-free silica layer of the nanoparticle is formed. In an example, the functionalized polyether molecules are functionalized PEG molecules and the polyether groups are PEG groups.

The solvent used in any of the steps in the method is an alcohol and an amount of water suitable to hydrolyze the precursors (e.g., silica precursors, FRM-conjugate precursors, and/or functionalized PEG molecules). In an embodiment, the solvent comprises alcohol and suitable amount of water. In an embodiment, the alcohol is ethanol.

Determining appropriate conditions for the core forming reaction, FRM-free silica layer forming reaction, FRM-containing silica layer reaction, or outermost FRM-containing silica layer reaction is within the purview of one having skill in the art. For example, the core forming reaction, FRM-free silica layer forming reaction, FRM-containing silica layer reaction, or outermost FRM-containing silica layer reaction are carried out at 10 °C to 30 °C, including all integer °C values and ranges therebetween, for 10 minutes to 120 minutes, including all integer minute values and ranges therebetween.

The base used in any of the steps in the method catalyzes a condensation reaction of the precursors (e.g., silica precursors, FRM-conjugate precursors, and/or functionalized PEG molecules). In an embodiment, the base is ammonia (e.g., ammonia in an aqueous solution).

In yet another aspect, the present disclosure provides uses of the multilayer, FRM-containing nanoparticles. The nanoparticles can be used for applications such as imaging methods (e.g., intracellular bioimaging methods), high throughput screening, medical diagnostics, sensing, and product tracking.

The multilayer, FRM-containing nanoparticles can be used to target and/or identify specific cell types (e.g., cells with certain internal structures), without lysing the cells. The methods use fluorescent microscopy or flow cytometry to detect distinct multilayer, FRM-containing nanoparticles. In an embodiment, an imaging method comprises the steps of: contacting a cell or plurality of cells with a plurality of multilayer, FRM-containing nanoparticles (or a plurality of distinguishable nanoparticles); and obtaining a plurality of images of the cell or plurality of cells, each image obtained using a different excitation wavelength and a different emission wavelength, where each different excitation wavelength is in the absorption spectrum a different type of FRM present in the nanoparticle and each different emission wavelength is in the emission spectrum of a different type of FRM present in the nanoparticle. The method may further comprise the step of combining the plurality of images to provide a single image. In the imaging methods, the image can be carried out using fluorescent microscopy techniques such as, for example, confocal microscopy. It may be desirable to use nanoparticles having a plurality of moieties conjugated to the PEG groups.

For example, the imaging methods can be used to identify cells based on their interior structures in a multiplexed manner. In an embodiment, the cells are contacted with the nanoparticles or a plurality of distinguishable nanoparticles having a size of 1 nm to less than 100 nm such that the nanoparticles are taken into the cells. For example, the cells can be subjected to electroporation (e.g., square-wave electroporation) to facilitate incorporation of the nanoparticles into the cells.

The cell or plurality of cells may be present in a subject. The subject can be any subject having a cell or plurality of cells. For example, the subject can be a human or non-human animal.

The following examples are presented to illustrate the present disclosure. They are not intended to limiting in any manner.

## EXAMPLE 1

The following is an example describing synthesis and use of multilayer, FRM-containing nanoparticles of the present disclosure.

In this example, bright and optically encoded fluorescent core-shell silica nanoparticles were produced. The nanoparticles are referred to as *multicolor* Cornell dots or simply *mc*C dots and have sizes below 100 nm, which makes the nanoparticles desirable for high throughput screening and applying them to intracellular bioimaging using fluorescence multiplexing. These nanoparticles are encoded with three spectrally distinct organic fluorophores, i.e., N-(7-dimethylamino-4-methylcoumarin-3-yl)maleimide (DACm, $\lambda_{abs}$ = 395 nm, $\lambda_{em}$ = 450 nm), tetramethylrhodamine-5-maleimide (TMRm , $\lambda_{abs}$ = 540 nm, $\lambda_{em}$ = 570 nm) and Cy5-maleimide (Cy5m, $\lambda_{abs}$ = 640 nm, $\lambda_{em}$ = 670 nm), with three precisely controlled numbers of dyes, i.e. 0, 5 and 20, respectively, per particle, resulting in 26 optically distinguishable nanoparticles as shown in Figure 1. The dyes were chosen based on commonly available excitation laser sources ($\lambda_{exc}$ = 405 nm, 540 nm and 633 nm) used in confocal microscopy or in high throughput screening techniques like flow cytometry, thus rendering *mc*C dots useful for standard fluorescence instrumentation. The particle architecture is designed such that the dyes are added to a dye doped particle core in a layer-by-layer fashion and each spectrally distinct dye is spatially separated by a pure silica shell in order to reduce energy transfer between the dyes (Figure 1). This assures maximum brightness levels and thus maximum signal-to-noise ratios in imaging.

Optically encoded fluorescent silica nanoparticles having distinct fluorescent signatures were made, which can be used in the development for screening assays. The versatility of silica surface chemistry allowed the nanoparticle surface to be modified with biomolecular probes such as oligonucleotides or peptides. The number of fluorescent optical codes 'C' that can be generated from silica based *mc*C dots depends on two parameters, the number of dye colors, 'm', and the number of fluorescence intensity levels, 'N', associated with each color. For intensity levels ranging from 0 to infinity the number of codes is defined as $C = N^m - 1$. The present case of three intensity levels, N = 3, of three colors, m = 3, thus leads to 26 color codes.

In order to carry out the synthesis *of mc*C dots three issues were addressed: (1) identification of the appropriate fluorescent dyes, 'm', based on commonly available excitation laser line sources; (2) identification of the appropriate numbers of dyes that can be reproducibly incorporated into the particles in order to generate distinguishable fluorescence intensity levels, 'N', and (3) spatial separation of the spectrally distinct dyes within the particle to minimize energy transfer and hence fluorescence quenching in order to achieve maximum brightness levels. The three dyes chosen for developing *mc*C dots were DACm, TMRm and Cy5m, *vide supra.* The fluorescence intensity measured for a single particle and single color depends on the number of dyes incorporated in it. Assuming that in a typical *mc*C dots synthesis batch the number of dyes of a specific color is Poisson distributed (thus assuming that dye encapsulation is a purely stochastic process), in order to distinguish different intensity levels of different *mc*C dots the mean values of these Poisson distributions were chosen such that there is minimum overlap between the wings of the distributions (see Figure 2a). It is thus desirable to precisely control the dye incorporation in the particle and to have minimum energy transfer between dyes on increasing their encapsulation numbers in order to avoid fluorescence quenching. For a given core size on increasing the number of dye molecules, based on spatial proximity dyes tend to show energy transfer. Hence to minimize cross talk between the dyes here a layer-by-layer approach was used to construct particles with high dye encapsulation numbers. Previous C dots synthesis work revealed that 20-30 nm diameter core-shell silica nanoparticles typically incorporated about 5-7 dyes per particle core for a given dye concentration. Assuming dye incorporation is Poisson distributed, for particles with mean dye numbers per particle of 10 and 15, respectively, an overlap of $\sim$ 45% and $\sim$ 25%, respectively, with 5 dyes per particle batches are expected as shown in Figure 2a. In contrast particles with a mean number of 20 dyes per particle only show $\sim$ 3% overlap with a 5 dyes per particle batch. A similarly small overlap is expected for the Poisson distribution of a 5 dyes per particle batch with a particle batch that contains zero dyes of that color (Figure 2a). Based on these considerations we chose the three distinct levels of 0, 5, and 20 dyes per particles, respectively, to distinguish different intensity levels of the same color in *mc*C dots.

Figure 2b-i shows a schematic of the various layer-by-layer particle synthesis routes leading to the 26 distinct *mc*C dots. The notation used in this example to distinguish *mc*C dot color codes contains lower case letters to denote fluorescence intensity levels (m/h for medium/high, i.e. $\sim$5/20 dyes per particle) and capital letters to denote fluorescence colors (G/R/B for green/red/blue emission, respectively). Each sequence moves from the core to the outer shell. For example, a mGhRmB *mc*C dot has a medium green ($\sim$5 dyes) core, followed by a high red ($\sim$20 dyes) inner shell, followed by a medium blue ($\sim$5 dyes) outer shell. A color not present (i.e. zero dyes of that color) in a *mc*C dot is denoted simply by omitting its notation. For example, a hGhB *mc*C dot has a high green ($\sim$20 dyes) core, no red (i.e. zero red dyes), and a high blue ($\sim$20 dyes) outer shell. Particle synthesis was carried out by first conjugating commercially available maleimide activated dye (TMRm, Cy5m, or DACm) to 3-mercaptopropyl-trimethoxysilane (MPTMS) using the maleimide-mercapto bioconjugation reaction to form dye conjugate (Figure 2b). Three single color particles containing medium dye loadings (mG, mR, or mB, respectively, see Figure 2c-e) were first synthesized via a modified Stöber-type silica condensation, by co-condensing the dye conjugate with TEOS at appropriate ammonia and water concentrations in alcohol. To this a thin silica shell was added by dosing the reaction solution with TEOS at concentrations below the nucleation threshold to avoid any secondary nucleation. A specific volume was removed from each reaction solution and these particles were set aside as particles with medium dye loadings. In order to get to the three single color particles with high dye loadings (hG, hR, or hB, respectively, see Figure 2c-e), to the remainder of the reaction solutions, dye conjugate was added to co-condense with the silanol groups on the surface of the core-shell silica nanoparticles. Following this step a thin silica shell was again added as described above. This alternating dye layer-silica shell procedure was carried out until the appropriate high dye loading with $\sim$ 20 dyes per particle was obtained. Figure 2c-e shows a schematic of the resulting onion-like structures for high green (TMRm), high red (Cy5m) and high blue (DACm) single color C dot particles. The necessary numbers of additional dye and silica shell layers on the core per particle to achieve high intensity emission were four for high green (TMRm) and high blue (DACm), and three for high red (Cy5m) particles.

Following the development of synthesis protocols to precisely control the number of dyes per particle for the three distinct dye systems we carefully devised particle architectures to incorporate all three dyes in the same particle with different dye loading levels for each dye. These particles were synthesized such that the three dyes were added in a layer-by-layer fashion with TMRm dye (green) in the core followed by Cy5m dye conjugate (red) in an inner shell followed by DACm dye conjugate (blue) added as the final dye layer (see center particle in Figure 1a). Organic fluorophores have relatively broad absorption and emission spectra. Depending on the spectral and spatial proximity of the various dyes in our particles, Förster resonance energy transfer is expected to occur. Such non-radiative energy transfer is the result of spectral overlap between the emission of a donor dye with the absorption spectrum of an acceptor dye. The efficiency of energy transfer between two dyes is determined by the Förster radius which is the distance at which 50% efficient energy transfer occurs between the donor and the acceptor. The calculated Förster radius for the pair DACm-TMRm is $\sim$ 40 Å (4.0 nm), while that of the pair TMRm-Cy5m is $\sim$ 45 Å (4.5 nm). Since DACm and Cy5m are on the extreme ends of the visible spectrum, with 15 Å (1.5 nm) the Förster radius of this pair is the smallest. The efficiency of energy transfer drops dramatically, as $1/r^6$, with separation distance, r, between the donor and the acceptor molecules. In order to effectively suppress energy transfer the TMRm and Cy5m dye layers in our particles were separated by a 10-12 nm thick silica shell (e.g. see Figure 2f). For particles containing no Cy5m dye, a silica shell thickness of 10-12 nm was grown on TMRm containing particle cores prior to DACm dye addition (e.g. see Figure 2g). For the first DACm dye layer addition to particles containing TMRm and Cy5m layers, a pure silica shell of 6-8 nm thickness was first grown (e.g. see Figure 2i). Hence, dye layers of different colors were spatially separated by thick enough silica shells to expect effective suppression of energy transfer.

Since TMRm was the dye chosen to be in the center, to maintain consistency in the synthesis of different *mc*C dots, a large batch of medium and high dye loaded TMRm particles were synthesized. The next step to obtain dual-color C dots was to grow a 10-12 nm thick silica separation shell on these nanoparticles followed by the addition of Cy5m dye conjugate (Figure 2f). After adding a final silica shell, this accounted for particles with medium Cy5m (mR) dye loadings. Following this step, two additional Cy5m dye layers plus their silica shells were added to obtain high Cy5m (hR) dye loaded particles. The reduction in additional layers to reach high dye loadings in larger *multicolor* C dots is due to their increased size relative to single color C dots providing more surface area per particle for additional dye attachment. Syntheses following these protocols provided the following four dual-color C dots with zero DACm dyes per particle: mGmR, mGhR, hGmR and hGhR. In order to obtain dual-color particles with zero Cy5m dyes per particle, DACm dye conjugate was added to mG and hG particles with a silica separation layer of 10-12 nm thickness to separate the TMRm and DACm dye layers (Figure 2g). This accounted for particles with medium DACm (mB) dye loadings. Three additional DACm dye layers and their silica shells were added to mGmB and hGmB particles in order to obtain high DACm (hB) dye loadings. Syntheses following these protocols provided the following four dual-color C dots with zero DACm dyes per particle: mGmB, mGhB, hGmB and hGhB.

Dual-color C dots with zero TMRm dyes per particle were synthesized using Cy5m core-shell particles as the template (Figure 2h). To that end a large batch of medium Cy5m doped particles was synthesized which was split into two portions, leaving one batch as particles with medium Cy5m (mR) dye loading, while to the other batch three alternating Cy5m dye and silica shell layers were added to obtain high Cy5m (hR) dye loaded particles (Figure 2d). A silica separation shell of 6-8 nm thickness was grown on these mR/hR nanoparticles followed by the addition of DACm dye conjugate (Figure 2h). Together with a final silica shell this accounted for particles with medium DACm (mB) dye loadings. Three alternating DACm dye and silica shell layers were subsequently added to obtain high DACm (hB) dye loadings. Syntheses following these protocols provided the final four dual-color C dots with zero TMRm dyes per particle: mRmB, mRhB, hRmB and hRhB.

Finally, in order to obtain triple color particles with all three dyes incorporated, a 6-8 nm thick silica shell was grown on mGmR, mGhR, hGmR and hGhR particles, followed by the addition of a DACm dye layer with silica shell. To the resulting mGmRmB, mGhRmB, hGmRmB and hGhRmB particles three additional DACm dye and silica shell layers were added to obtain high dye doped DACm (hB) particles (Figure 2i). Syntheses following these protocols provided the following eight triple color *mc*C dots: mGmRmB, hGmRmB, mGhRmB, hGhRmB, mGmRhB, hGmRhB, mGhRhB and hGhRhB.

The ability to precisely tune the dye numbers in such multicolor fluorescent silica nanoparticles comes with a considerable amount of architectural complexity. For example, triple color C dots with high dye loadings for TMRm, Cy5m and DACm (hGhRhB) contain four TMRm dye and four silica shell layers added to the medium TMRm dye loaded core-shell particle (adding up to nine layers around the TMRm core), followed by three dye and three silica shell layers to obtain the high Cy5m dye loading, followed by four dye and four silica shell layers to obtain the high DACm dye loading. All particles were finally surface coated with a polyethylene glycol (PEG) layer to provide steric stabilization in buffer solutions and to render them more biocompatible. The result is onion-type architecture with 24 distinct layers (including the PEG layer) around a dyed core, of which 11 are dye layers and 12 are pure silica shell layers. Based on the various dye per particle combinations 26 spectroscopically distinguishable particles were synthesized as shown in Figure 1. Figure 1a displays color renderings of these 26 particles assembled in a way reflecting the synthesis pathways described above. Figure 1b depicts a photo of the 26 particles in aqueous solutions in cuvettes under ambient light. Cuvettes are organized according to green TMRm dye loadings, with particles containing no, medium, and high TMRm dye loadings sitting in the bottom, middle, and upper rows, respectively (see Figure 4b for specific assignments). For comparison, the 27th cuvette on the bottom left of Figure 1b is filled with non-dyed PEGylated silica nanoparticles.

Experimental Section. Chemicals and materials. To carry out particle synthesis all chemicals were used as received. Tetraethoxysilane (TEOS, $\geq$ 99%, GC) and ammonia in ethanol (2.0 M) were purchased from Sigma Aldrich. (3-mercaptopropyl)-trimethoxysilane (MPTMS, >96% purity, Gelest Inc.) was used as a conjugation linker for dye incorporation in silica nanoparticles. The dyes used for the nanoparticle syntheses were Cy5-maleimide (Cy5m, GE Healthcare Life Sciences), tetramethylrhodamine-5-maleimide (TMRm, Life Technologies) and N-(7-dimethylamino-4-methylcoumarin-3-yl)maleimide (DACm, Anaspec, Inc.). The dyes were dissolved in dimethyl sulfoxide (DMSO, anhydrous $\geq$ 99.9%, Sigma Aldrich). Ethoxy-silane terminated poly-(ethylene glycol) (mPEG-Silane, molar mass ~5000 g/mol) was purchased from Layson Bio. The reactions were carried out in ethanol (200 proof, Pharmaco-Aaper) and deionized water (DI water, 18.2 M$\Omega$.cm$^{-1}$, purity, Millipore Milli-Q system). The particles were dialyzed using 10,000 molecular weight cut off (MWCO) Snakeskin dialysis membrane tube (Pierce) and were filtered with 0.2 $\mu$m PTFE syringe filters (Fisher Scientific). The particles were transferred into Dulbecco's Phosphate-Buffered Saline 1X buffer (DPBS without calcium and magnesium, Life Sciences) to carry out cell measurements using Macrosep® Advance Centrifugal Device, with a MWCO 30,000 (Pall Corporation). Sodium azide (BioUltra, $\geq$ 99.5%, Sigma Aldrich) was added to the particles in buffer solution to act as biocide. For cellular imaging, the nanoparticles were electroporated using Gene Pulser X (Bio-Rad). The surface of the cells was labeled with Alexa Fluor 488 cholera toxin subunit B (Invitrogen).

Nanoparticle Syntheses. Dye conjugation. The maleimide derivative of the dyes TMRm (2.6 mM in DMSO), Cy5m (1.26 mM in DMSO) and DACm (3.4 mM in DMSO) were dissolved in DMSO in nitrogen atmosphere glove box for 15 hours. For nanoparticle syntheses the dyes were conjugated with MPTMS in 1:25 dye to silane molar ratio in the glove box for 10-12 hours. Concentrations of $10\times10^{-5}$ M/$4.0\times10^{-5}$ M/$45\times10^{-5}$ M were used for TMRm/Cy5m/DACm dye conjugates, respectively.

Syntheses of single color C dots with medium and high dye loadings. Medium and high TMRm loaded nanoparticle syntheses. To 10 ml ethanol solution containing 0.88 M DI water and 0.2 M ammonia in ethanol, $1.3\times10^{-5}$ M TMRm dye conjugate was added and left to stir for 15 minutes. To this solution 0.055 M TEOS was added and the reaction was left to stir for 12 hours. To this solution 0.105 M neat TEOS was added drop wise at a rate of 1 μl per ml of reaction volume every 30 minutes resulting in medium TMRm loaded particles (mG).

For high TMRm loaded particles (hG), $2\times10^{-5}$ M TMRm dye conjugate was added to 5 ml of mG reaction solution and left to stir for 8 hours, followed by addition of 0.105 M neat TEOS at a rate of 1 μl per ml of reaction volume every 30 minutes. The solution was left to stir for 4 hours before further TMRm dye layer-TEOS silica shell addition. This sequence was repeated a total of four times to obtain hG particles.

Medium and high Cy5m nanoparticle syntheses. To 10 ml ethanol solution containing 0.88 M DI water and 0.2 M ammonia in ethanol, $1\times10^{-5}$ M Cy5m dye conjugate was added and left to stir for 15 minutes. To this solution 0.055 M TEOS was added and the reaction was left to stir for 12 hours. To this solution 0.105 M neat TEOS was added drop wise at a rate of 1 μl per ml of reaction volume every 30 minutes resulting in medium Cy5m loaded particles (mR).

For high Cy5m loaded particles (hR), $1\times10^{-5}$ M Cy5m dye conjugate was added to 5 ml of mR reaction solution and left to stir for 8 hours, followed by addition of 0.105 M neat TEOS at a rate of 1 μl per ml of reaction volume every 30 minutes. The solution was left to stir for 4 hours before further Cy5m dye layer-TEOS silica shell addition. This sequence was repeated a total of three times to obtain hR particles.

Medium and high DACm nanoparticle synthesis. To 10 ml ethanol solution containing 0.88 M DI water and 0.2 M ammonia in ethanol, $5\times10^{-5}$ M DACm dye conjugate was added and left to stir for 15 minutes. To this solution 0.055 M neat TEOS was added and the reaction was left to stir for 12 hours. To this solution 0.15 M neat TEOS was added drop wise at a rate of 1 μl per ml of reaction volume every 30 minutes resulting in medium DACm loaded particles (mB).

To 5 ml of mB particle solutions, 5 ml of ethanol was added to synthesize high DACm loaded particles (hB). The concentrations of DI water and ammonia in ethanol were maintained at 0.88 M and 0.2 M, respectively. $9\times10^{-5}$ M DACm dye conjugate was added to the 10 ml reaction solution and left to stir for 8 hours, followed by 0.15 M neat TEOS at a rate of 1 μl per ml of reaction volume every 30 minutes. The solution was left to stir for 4 hours before further DACm dye layer-TEOS silica shell addition. This sequence was repeated a total of four times to obtain hB particle solutions.

Syntheses of multicolor C dots (mcC dots). Silica shell synthesis on medium and high TMRm loaded particles: Medium TMRm loaded particles (mG) were synthesized by scaling the reaction solution up to 100 ml. $1.3\times10^{-5}$ M TMRm dye conjugate was added to 100 ml of ethanol containing 0.88 M DI water and 0.2 M ammonia in ethanol and left to stir for 15 minutes, followed by 0.055 M neat TEOS and was left to stir for 12 hours. To this solution 0.105 M neat TEOS was added drop wise at a rate of 1 μl per ml of reaction volume every 30 minutes to form a silica shell. Following the mG particle synthesis the solution was separated into two round bottom flasks with 50 ml each. To one of the 50 ml mG particle solution, $2\times10^{-5}$ M TMRm dye conjugate was added and left to stir for 8 hours, followed by addition of 0.105 M neat TEOS at a rate of 1 μl per ml of reaction volume every 30 minutes. The solution was left to stir for 4 hours before further TMRm dye layer-TEOS silica shell addition. This sequence was repeated a total of four times to obtain high TMRm particles (hG).

To each of the 50 ml mG and hG solutions, 2.6 M neat TEOS was added at a rate of 2 μl per ml of reaction volume every 30 minutes to get a 10-12 nm thick silica shell before carrying out dual color particle synthesis.

Silica shell synthesis on medium and high Cy5m loaded particles. Medium Cy5m loaded particles (mR) were synthesized by scaling to 30 ml reaction system. $1\times10^{-5}$ M Cy5m dye conjugate was added to 30 ml ethanol solution containing 0.88 M DI water and 0.2 M ammonia in ethanol and left to stir for 15 minutes followed by 0.055 M neat TEOS and the reaction was left to stir for 12 hours. To this solution 0.105 M neat TEOS was added drop wise at a rate of 1 μl per ml of reaction volume every 30 minutes to form a silica shell. The mR particle solution was separated into two round bottom flasks with 15 ml each. To one of the 15 ml mR particle solution, $1\times10^{-5}$ M Cy5m dye conjugate was added and left to stir for 8 hours, followed by addition of 0.105 M neat TEOS at a rate of 1 μl per ml of reaction volume every 30 minutes. The solution was left to stir for 4 hours before further Cy5m dye layer-TEOS silica shell addition. This sequence was repeated a total of three times to obtain high Cy5m loaded particles (hR).

To each of the 15 ml mR and hR solutions, 1.8 M neat TEOS was added at a rate of 2 μl per ml of reaction volume every 30 minutes to get a shell that was 6-8 nm thick.

Syntheses of dual color C dots. Cy5m dye conjugate addition to medium and high TMRm particles. To synthesize particles with Cy5m dye as the second layer, 30 ml of mG and hG particle solutions with 10-12 nm thick silica shell were taken in two separate round bottom flasks. For medium Cy5m loading, $1\times10^{-5}$ M Cy5m dye conjugate was added to each of the reaction flasks containing 30 ml of mG and hG particle solutions and left to stir for 8 hours. To this reaction solution 0.105 M neat TEOS was added drop wise at the rate of 1 μl per ml of reaction volume every 30 minutes to form a thin silica shell. 15 ml was removed from each reaction solution and was set aside as mGmR and hGmR particle solutions. For high Cy5m loading, $1 \times 10^{-5}$ M Cy5m dye conjugate was added to each of 15 ml mGmR and hGmR reaction solutions and left to stir for 8 hours, followed by addition of 0.105 M neat TEOS at a rate of 1 μl per ml of reaction volume every 30 minutes. The solution was left to stir for 4 hours before further Cy5m dye layer-TEOS silica shell addition. This alternating Cy5m dye conjugate-TEOS shell addition sequence was carried out a total of two times in order to get particles with mGhR and hGhR particle solutions.

DACm dye conjugate addition to medium and high TMRm particles. To synthesize particles with DACm as the second dye layer, 10 ml of the medium and high TMRm particle solutions with 10-12 nm thick silica shell were taken in two separate round bottom flasks (from section 1.3.a).

For medium DACm loading, $5 \times 10^{-5}$ M DACm dye conjugate was added to each of the reaction flasks containing 10 ml of mG and hG particle solutions and the reaction solution was left to stir for 8 hours. A thin silica shell was added to the reaction by drop wise addition of 0.15 M neat TEOS at a rate of 1 μl per ml of reaction volume every 30 minutes to form silica shell. 5 ml of the reaction solutions were set aside as mGmB and hGmB particle solutions.

For high DACm loading, 5 ml of ethanol was added to 5 ml of dual color mGmB and hGmB particle solutions. The concentrations of DI water and ammonia in ethanol were maintained at 0.88 M and 0.2 M, respectively. $9 \times 10^{-5}$ M DACm dye conjugate was added to the 10 ml reaction solution and left to stir for 8 hours, followed by 0.15 M neat TEOS at a rate of 1 μl per ml of reaction volume every 30 minutes. The solution was left to stir for 4 hours before further dye layer-TEOS silica shell addition. This alternating DACm dye conjugate-TEOS shell addition sequence was carried out a total of three times to get high DACm loaded particles as mGhB and hGhB particle solutions.

DACm dye conjugate addition to medium and high Cy5m particles. To synthesize particles with DACm as the second dye layer, 10 ml of the mR and hR particle solutions with 6-8 nm thick silica shell were taken in two separate round bottom flasks (from section 1.3.b).

For medium DACm loading, $5 \times 10^{-5}$ M DACm dye conjugate was added to each of the reaction flasks containing 10 ml of mR and hR particle solutions and the reaction solution was left to stir for 8 hours. A thin silica shell was added to the reaction by drop wise addition of 0.15 M neat TEOS at a rate of 1 μl per ml of reaction volume every 30 minutes to form silica shell. 5 ml of the reaction solutions were set aside as mRmB and hRmB particle solutions.

For high DACm loading, 5 ml of ethanol was added to 5 ml of dual color mRmB and hRmB particle solutions. The concentrations of DI water and ammonia in ethanol were maintained at 0.88 M and 0.2 M, respectively. $9 \times 10^{-5}$ M DACm dye conjugate was added to the 10 ml reaction solution and left to stir for 8 hours, followed by 0.15 M neat TEOS at a rate of 1 μl per ml of reaction volume every 30 minutes. The solution was left to stir for 4 hours before further dye layer-TEOS silica shell addition. This alternating DACm dye conjugate-TEOS shell addition sequence was carried out a total three times to get high DACm loaded particles as mRhB and hRhB particle solutions.

Syntheses of triple color C dots. Silica shell syntheses on dual color particles containing TMRm and Cy5m. To 10 ml of mGmR, mGhR, hGmR and hGhR as mentioned in section 1.3.c., 1.8 M neat TEOS was added at a rate of 2 μl per milliliter of reaction volume every 30 minutes to get a shell that was 6-8 nm thick. 10 ml solution of each of dual color particles containing TMRm and Cy5m were taken in separate round bottom flasks. The concentrations of DI water and ammonia were maintained at 0.88 M and 0.2 M, respectively.

DACm dye conjugate addition to dual color particles loaded with TMRm and Cy5m. For medium DACm loading, $5 \times 10^{-5}$ M DACm dye conjugate was added to each of the reaction flasks containing 10 ml of mGmR, mGhR, hGmR and hGhR particle solutions and was left to stir for 8 hours. A thin silica shell was added to the reaction by drop wise addition of 0.15 M neat TEOS at a rate of 1 μl per milliliter of reaction volume every 30 minutes to form silica shell. 5 ml of the reaction solutions were set aside as mGmRmB, mGhRmB, hGmRmB and hGhRmB particles.

For high DACm loading, 5 ml of ethanol was added to 5 ml of triple color particle solutions containing medium DACm loaded particles. The concentrations of DI water and ammonia in ethanol were maintained at 0.88 M and 0.2 M, respectively. $9 \times 10^{-5}$ M DACm dye conjugate was added to the 10 ml reaction solution and left to stir for 8 hours, followed by 0.15 M neat TEOS at a rate of 1 μl per milliliter of reaction volume every 30 minutes. The solution was left to stir for 4 hours before further DACm dye layer-TEOS silica shell addition. This alternating DACm dye conjugate-TEOS shell addition sequence was carried out three times to get high DACm loaded particles: mGmRhB, mGhRhB, hGmRhB and hGhRhB particles.

Silica shell growth on nanoparticles prior to PEGylation. A silica shell was grown on all particles by drop wise addition of TEOS to get the same size for all the nanoparticles. For all solutions the concentrations of DI water and ammonia in ethanol were maintained at 0.88 M and 0.2 M, respectively.

To 10 ml solution containing medium (section 1.1.b)/high (section 1.1c) DACm particles, 4.8 M TEOS was added and to medium/high TMRm particles (section 1.3.a), 2.7 M TEOS was added at a rate of 2 μl per milliliter of reaction volume every 30 minutes. To 5 ml of medium and high Cy5m particle solutions (section 1.3.b), 3.6 M TEOS was added at a rate of 2 μl per milliliter of reaction volume every 30 minutes.

To 5 ml of the dual color particle solutions containing G-R (section 1.3.c)/ G-B (section 1.3.d)/R-B (section 1.3.e), 2.6 M TEOS/ 2.6 M TEOS/ 3.3 M TEOS were added, respectively, at a rate of 2 μl per milliliter of reaction volume every 30 minutes.

To 5 ml of the triple color particle solutions containing only medium DACm particles (section1.3.f), an additional 0.7 M TEOS was added at a rate of 2 μl per milliliter of reaction volume every 30 minutes. No additional TEOS was added to triple color particles containing high DACm.

PEGylation *of mc*C dots nanoparticles. To 1.0 liter of DI water was adjusted to pH 5 by adding 2.0 M hydrochloric acid (aq.) using a pH meter (VWR International Symphony, SB70P). To carry out PEGylation, all the particles were grown to the same size as mentioned in the particle synthesis section. 0.08 M aqueous PEG-silane (mPEG-silane, M.W. 5k) solution was made by dissolving the PEG-silane in DI water at pH 5. 1 ml of this solution was taken in a vial and 2 ml of ethanol was added and the solution was left to stir for about 10 minutes. 1.0 ml of the as-made particle solution was added drop wise to 3 ml of mPEG-silane dissolved in the ethanol-water mixture, and then the solution was left to stir in an oil bath maintained at 70°C for 24 hours.

After PEGylation the particles were dialyzed in DI water using 10,000 MWCO dialysis membrane tube and filtered with 0.2 μm PTFE syringe filters and stored in dark at room temperature for further characterization.

For cell imaging measurements the particles were transferred into a Dulbecco's Phosphate-Buffered Saline *(DPBS)* using Macrosep® Advance Centrifugal Device, with a MWCO of 30,000. Following dialysis, 5 ml of the PEGylated nanoparticle solution was added to 5 ml of buffer solution and centrifuged for 30 minutes at 3500 rpm. This process was repeated three times with buffer solution. As a final step the particles were heated to 75°C for 15 minutes to pasteurize the solution followed by the addition of 0.1 % (w/v) sodium azide aqueous solution.

Characterization of medium and high dye loaded nanoparticles. Photographs of the reflected light image of the 27 cuvettes were taken under ambient light. The photo image was arranged as particles with no TMRm dye (bottom row), particles with ∼ 5 TMRm dyes (middle row) and particles with ∼ 20 dyes in the core (top row). Each row was taken separately using the identical settings of ISO 100 and 1/20th of a second exposure at f/4.5 on a Canon EOS digital Rebel 400 D camera, fixed on a tripod and was then stacked in the arrangement as mentioned using Adobe Photoshop.

Spectrometry and Spectrofluorometry. The particles were absorbance matched to the respective free dye by diluting the particles or free dye with DI water in a quartz cuvette using a Varian Cary 5000 Spectrometer (Varian, Palo Alto, CA). The extinction coefficients of DACm ($25,000 M^{-1}cm^{-1}$), TMRm ($98,000 M^{-1}cm^{-1}$) and Cy5m ($250,000 M^{-1}cm^{-1}$) were used to quantify the concentration of dyes in the samples. Fluorescence measurements of absorbance matched samples were performed on a Photon Technologies International Quantamaster Spectrofluorometer (PTI, Birmingham, NJ).

Fluorescence Correlation Spectroscopy (FCS). To quantify the brightness per particle, hydrodynamic radius and concentration of particles; the absorbance matched samples were measured on a home-built multi-spectral Fluorescence Correlation Spectroscopy (FCS) set up using solid state 405 nm (for DACm particles), HeNe 535 nm (for TMRm particles) and HeNe 633 nm (for Cy5m particles) laser excitation sources. The excitation beam was reflected through a 60X Olympus UPlan SAPO, 1.2NA water immersion objective. A 200μL volume at nanomolar concentrations of the fluorescent sample was placed on a microwell dish with a No. 1.5 coverslip bottom (MatTek P35G-1.5-10-C). The emitted light from the sample was collected by the objective, passed through the excitation/emission dichroic and was reflected into a focusing lens by the emission mirror. The emission light was focused though a long pass filter (Chroma) to remove any excitation light and collect only the emission photons. A 50 micron pinhole was used to axially limit the effective volume from which fluorescence was collected. The light then passed through a second lens into an avalanche photodiode (SPCM 14, Perkin Elmer). The resulting photocurrent was digitally autocorrelated with a correlator card (Correlator.com).

The data was fit using a triplet corrected autocorrelation function, as shown in the analytical form in equation 1.

$$G(\tau) = 1 + \left(\frac{1}{N}\right) \times \left(1 - A + A \times \exp\left(\frac{t}{\tau_R}\right)\right) \times \left(\frac{1}{\left(1 + \frac{t}{\tau_D}\right)}\right) \times \left(\frac{1}{\left(\sqrt{1 + \frac{t}{\tau_D} s^2}\right)}\right)$$

Equation 1

$A$ is the amplitude of triplet correction, $\tau_R$ is the apparent diffusion time of a dye molecule/particle in the triplet state, $\tau_D$ is the diffusion time of the molecule/particle in the singlet state and $N$ is the number of molecules in the focal volume. The structure factor parameter '*s*' describes the 3-D Gaussian focal volume in terms of the ratio of the axial to the radial axis and is calculated from measurements of the standard dye with known diffusion coefficient. To obtain the structure factor '*s*' for the 405 nm, 535 nm and 633 nm laser lines the dyes used were N-(7-dimethylamino-4-methylcoumarin-3-yl)-maleimide, tetramethylrhodamine-5-maleimide and Alexa Fluor 647-maleimide, respectively.

Cellular imaging. Cell culture. RBL-2H3 cells were maintained in monolayer culture in MEM supplemented with 20% FBS (Atlanta Biologicals) and 10 $\mu$g/ml gentamicin sulfate as previously described.

Nanoparticle delivery into cells via electroporation. Cells were harvested 3-5 days after passage and $1 \times 10^6$/ml RBL-2H3 cells were electroporated in 0.5 ml of cold electroporation buffer (137 mM NaCl, 2.7 mM KCl, 1 mM MgCl$_2$, 1 mg/ml glucose, 20 mM HEPES (pH 7.4) with 200$\mu$l of nanoparticles suspended in DPBS buffer (5-10 $\mu$M). Square wave electroporation was used with settings of 280 V, 10.0 ms pulse length, 4 pulses, and pulse intervals of 0.1 ms using Gene Pulser X (Bio-Rad). This procedure was repeated for each of the 26 nanoparticles. Electroporated cells were allowed to recover for approximately an hour before being fixed with 4% paraformaldehyde and 0.1% glutaraldehyde. Fixed cells were then labeled with 0.5 $\mu$g/ml Alexa 488 cholera toxin subunit B for 15 min at room temperature to stain the cell membrane.

Confocal imaging. Cells containing nanoparticles were imaged on a Zeiss 510 LSM confocal microscope using a 40X water objective (N.A=1.2). Images were taken sequentially using the 405, 488, 561, 633 nm laser lines for excitation and the 420-480 (Band Pass, BP), 575 (Long Pass, LP), 505-550 (BP) and 650 (LP) filter sets for collecting the emitted light. ImageJ was used to split the images into green, red, blue, yellow and brightfield images. The overlaid images were concatenated in ImageJ, containing only images from the four emission channels. The particles were identified based on colocalization of the red, green and blue images.

All resulting particles were PEGylated using 5k molar mass poly(ethylene glycol)-silane before carrying out any spectroscopic measurements (see experimental section). Since a description of the full spectroscopic characterization of all 26 particle species is beyond the scope of this paper, here only representative examples for specific *mc*C dots are provided, together with a summary of the spectroscopic measurements of brightness levels of all colors in all 26 particles, see Figures 3 and 4. In order to characterize the nanoparticles and understand their fluorescent properties, first aqueous solutions of the medium and high DACm, TMRm, and Cy5m dye loaded single color nanoparticles were absorbance matched to those of the respective parent free dyes. Figure 3a-c shows the resulting absorbance and emission spectra. No significant spectral shifts between free dyes and particles were observed, suggesting that the electronic structure of the dyes were preserved upon encapsulation. The medium and high DACm, TMRm and Cy5m dye doped nanoparticles show an enhancement of $\sim$ 6.0, $\sim$1.3, and $\sim$1.5, respectively, relative to free dye. For all three systems dye encapsulation into the rigid silica environment thus leads to significant brightness enhancements relative to free dye in aqueous solution, consistent with earlier studies and suggesting that under these conditions the dyes don't exhibit fluorescence quenching even at the high loading levels demonstrated here.

The number of dyes incorporated into the particles was quantified using fluorescence correlation spectroscopy (FCS) in combination with absorbance measurements. The FCS experimental set up was equipped to excite dyes at $\lambda$ = 405nm, 543 nm and 633 nm, respectively (see experimental section). FCS is a diffusion based spectroscopy technique using the fluorescence of the diffusing moiety to generate an autocorrelation curve. Similar to dynamic light scattering (DLS), the time scale on which the correlation decays can be related to a diffusion coefficient which in turn can be related to a hydrodynamic radius/diameter of the diffusing moiety. Figure 3d-f compares the autocorrelation curves of the three parent dyes (solid lines) with those of the six single color C dots (mG, mR, and mB, open symbols; hG, hR, and hB, closed symbols) derived from the three dyes/colors at medium and high dye loadings, respectively. Dye incorporated nanoparticles diffuse much slower than the parent free dyes, confirming a significant increase in hydrodynamic radius from free dye to particle. Furthermore, medium and high dye level containing particles show very similar correlation curves revealing the high control over the final targeted particle size. Analysis of the autocorrelation curves for the DACm, TMRm and Cy5m systems reveals diameters of 1.3 nm, 1.4 nm and 1.4 nm, respectively, for the free dyes, as well as 40 nm, 45 nm and 35 nm, respectively, for both medium and high dye loaded nanoparticles.

From the amplitude, G(0), of the FCS autocorrelation curve one can obtain the number of fluorescent moieties in the focal volume from which their concentration can be deduced. Using this particle concentration along with the concentration of dyes obtained from the absorbance measurements one can calculate the number of dyes per particle. This is a piece of information which allowed quantitative assessments of dyes per particle numbers in the synthesis of *multicolor* C dots (*mc*C dots) and was used as feedback to optimize synthesis protocols to achieve the necessary levels of control described herein. Based on these measurements we calculated the number of dyes per particle for medium and high dye loaded particles to be 6/27, 6/22 and 6/24 for TMRm, Cy5m and DACm dye loadings. Furthermore, from the product of the fluorescence enhancement of a dye encapsulated in the particle over free dye in aqueous solution and the number of dyes per particle, a brightness factor for each dye-particle system can be calculated. This factor describes how much brighter the particles are relative to a single free dye in aqueous solution. The respective brightness factors determined in this way were: 9.1/35 for the medium/high TMRm particles, 9/33 for the medium/high Cy5m particles, and 36/144 for the medium/high DACm particles. These results suggest that the particles should be one to two orders of magnitude brighter than the parent free dyes. Besides their multiplexing properties, these very high brightness levels will make *mc*C dots very attractive for bioimaging applications. The calculated brightness factors can be compared to the experimental brightness of the dyes and particles as measured by the count rates of the individual diffusing species on the optical FCS detector. This provides a direct measure of the brightness of the free dyes and particles, respectively. Figure 3g-i show the results of these measurements. Figure 3g shows that medium/high TMRm loaded particles are 6.6 ($\pm$ 0.1)/32 ($\pm$ 0.4) times brighter than parent TMRm free dye, while for the Cy5m system, Figure 3h shows that medium/high Cy5m loaded particles are 5.3 ($\pm$ 0.2)/27.6 ($\pm$ 0.4) times brighter than the parent Cy5m free dye. Figure 3i compares the brightness for the DACm particle system and shows medium/high DACm loaded particles to be 24 ($\pm$ 3.0)/105 ($\pm$ 10) times brighter than parent DACm free dye, respectively. These direct brightness measurements confirm that as targeted, individual brightness levels are apart from one another by a factor of 4-6. Consistent with similar observations in earlier studies, the calculated brightness factors systematically overestimate the values obtained from FCS measurements. This may be due to an error in the determination of dye equivalents, which e.g. assumed no change in absorption cross section between free and encapsulated dyes. It may further be due to the fact that FCS measurements were carried out with polarized light, which possibly only excited sub-sets of the dyes in the particles, in turn leading to smaller counts per particle than expected from the brightness factor.

Previous work on fluorescent multi-shell nanoparticles with sizes > 3 $\mu$m revealed that the fluorescence intensity of the particles decreased on increasing the number of shell layers, due to changes in refractive index causing scattering of the emitted light. We checked whether the same effect applied to the present particles by comparing the ratios of the number of dyes per particle to the brightness per particle for medium and high dye loaded particles. Our results showed that for medium and high DACm loaded particles the ratio was 4.0/4.3, for medium and high TMRm loaded particles the ratio was 4.5/4.8, and for medium and high Cy5m loaded particles the ratio was 3.66/3.69. These results reveal that in contrast to previous results in our system moving from medium to high dye loadings by incorporating additional silica shells does not decrease the relative fluorescence emission.

Figure 4a shows representative FCS curves of specific particles moving towards triple color *mc*C dots covering the entire synthetic scheme starting from medium/high TMRm dye loaded single color C dots (mG, hollow circles/hG, full circles) via medium/high Cy5m dye loaded dual-color C dots (hGmR, hollow circles/hGhR, full circles) to the final triple color *mc*C dots with medium/high DACm dye loadings (hGhRmB, hollow circles/hGhRhB, full circles). All particles in this plot were terminated using a PEGylation step, although they were not grown to the same size of ~ 85 nm. Results clearly indicate the increase in the particle size as reflected in a shift of the correlation curves to longer times on starting from medium TMRm dye loaded particles to particles that contain all three dyes at high dye loadings. FCS curves of green fluorescent particles show pronounced contributions from triplet states at short times, which was accounted for by the fitting procedure (see experimental section). Medium TMRm loaded core shell particle synthesis (mG, hollow green circles), resulted in particles of 15 nm ($\pm$ 0.5) diameter (including the PEG layer). Addition of four TMRm dye and silica shell layers resulted in 28 nm ($\pm$ 0.6) diameter (including the PEG layer) high TMRm dye loaded particles (hG, solid green circles). Each of the four alternating dye and silica shell layers contributed 1.2-1.5 nm to the shell thickness (or 2.4-3 nm to the particle diameter). Dual-color particles with medium Cy5m dye loading first had a 10-12 nm thick silica shell (increasing the particle diameter by 20-24 nm) grafted onto the 28 nm diameter hG particles followed by the addition of one alternating Cy5m dye and silica shell layer (hGmR, hollow red circles). Comparison of the autocorrelation curves for hG and hGmR particles reveals a large difference in particle size as the hGmR curve is shifted significantly to the right of the hG curve. Analysis reveals a diameter of 53 nm ($\pm$ 2.0) for the dual-color C dots. Particles with high Cy5m dye loading with two additional alternating Cy5m dye and silica shell layers on the hGmR particle resulted in 57 nm ($\pm$ 2.2) diameter dual-color C dots (hGhR, solid red circles). Each additional Cy5m dye and silica shell layer increased the thickness of the particle by ~ 1.0-1.1 nm (increasing the particle diameter by 2.0-2.2 nm). Finally, particles containing the third, DACm dye had a 6-8 nm thick silica separator shell (increasing the particle diameter by 12-16 nm) grown onto the 57 nm ($\pm$ 2.2) hGhR particles followed by the addition of a single alternating DACm dye and silica shell layer, producing triple color C dots (hGhRmB, hollow blue circles) with a particle diameter of ~ 72 nm ($\pm$ 4.1). The increase in the particle size is noticeable when comparing the FCS curves of hGhR and hGhRmB. The final FCS curve in Figure 4a is from triple color C dots with high loadings of all three dyes (hGhRhB, solid blue circles) with three additional alternating DACm dye and silica shell layers plus a PEG surface layer, added to the hGhRmB particles resulting in a final particle diameter of 85 nm ($\pm$ 4.6).

The absence of energy transfer between dyes of different color in the same particle, i.e. the efficiency of the pure silica layer to suppress Förster resonance energy transfer (FRET), is highlighted in Figure 4b showing six fluorescence emission spectra of solutions of four particles (hGhRhB, hGhRmB, mGhRhB and hGmRhB) each containing all three dyes/colors. For the spectra on the left and right (blue and red emission, respectively,) solutions were absorbance matched in the green, while for the spectra in the middle (green emission) solutions were absorbance matched in the red. Figure 4b compares emission profiles from excitations at 405 nm, 540 nm, and 633 nm, respectively, of medium and high DACm (mB/hB), TMRm (mG/hG), and Cy5m (mR/hR) containing triple color particles. Particles with high DACm dye loading (hGhRhB) were ~3.8 times brighter than with medium DACm loading (hGhRmB) when excited at 405 nm. Particles with high TMRm dye loading (hGhRhB) were ~3.5 times brighter than with medium TMRm loading (mGhRhB) when excited at 540 nm. On excitation at 633 nm, particles with high Cy5m loading (hGhRhB) were ~4.0 times brighter than with medium loading (hGmRhB). The very different emission levels confirm that particles containing medium and high dye loading for each color can clearly be spectroscopically differentiated. Furthermore, none of the spectra with blue or green emission show spectroscopic evidence for FRET. For example, on exciting samples hGhRmB or hGhRhB at 405 nm (in the blue) no significant energy transfer from DACm to TMRm or Cy5m is evident via green or red emission, respectively. Similarly, when exciting hGhRhB or mGhRhB particles at 540 nm, no significant energy transfer from TMRm to Cy5m is observed via red emission. We conclude that the silica shells used to spatially separate layers of different color dyes clearly suppress FRET that would otherwise be observed in the spectra.

Figure 4c summarizes the spectroscopic brightness levels of all colors in all 26 particles in the form of bar graphs positioned in the identical way to the cuvettes in Figure 1b filled with particle solutions. Brightness levels were measured as the respective fluorescence emission maxima for each color of each particle as exemplified in Figure 4b. The height of the bars for each color was normalized to the brightness of particles with medium dye loading. On going from top to bottom, in analogy to the photo in Figure 1b, we have separated the display into three families/rows with decreasing number of green TMRm dyes (green bars) with ∼20 (top row), ∼5 (middle row), and 0 dyes (bottom row) per particle in the core of the particles. From these bar graphs, the particles with high TMRm dye loading were ∼ 3.5 times brighter than the medium dye loaded TMRm particles. On going across the figure from left to right we compare the fluorescence brightness levels in each of these "green" families/rows for particles containing different amounts of Cy5m (red bars) and DACm dyes (blue bars). From the results, as targeted in the design (*vide supra*) particles with high Cy5m dye loading were ∼ 4-4.3 times brighter than particles containing medium Cy5m dye levels, whereas the high DACm loaded particles were about ∼ 3.8-4.2 times brighter than the medium DACm loaded particles. Measured particle brightness levels as revealed in Figure 4c for the three colors of *mc*C dots are thus solely due to the number of dyes of that color in the particle. This allows predictions of brightness levels based on synthesis protocols and renders identification of specific color codes more manageable.

Biological Multiplexing in Cell Assemblies. Following the characterization of *mc*C dots, fluorescence multiplexing was carried out to demonstrate multicolor intra-cellular imaging. Individual cell solutions were electroporated with one type of particle solution (see experimental section), following this the cells were mixed and differentiated based on the RGB color mixing scheme using confocal imaging. Rat basophilic leukemia mast cells (RBL-2H3) were used for these measurements and the cell surface was labeled with Alexa488-Cholera toxin subunit B ($\lambda_{abs}$ = 488 nm, $\lambda_{em}$ = 515 nm, a wavelength not utilized in *mc*C dot syntheses) to identify the cell periphery.

Figure 5 shows confocal fluorescence microscopy images of mixture of cells, containing hB, hGhB, hGhR and hGhRmB particles, respectively. Figure 5 (a-d) show cell images as obtained in green, red, blue and yellow channels, upon exciting the cells at 560 nm, 633 nm, 405 nm and 488 nm, respectively. These images show emission for each color channel corresponding to photons emitted from each dye color present in the nanoparticle. RBL-2H3 cells show autofluorescence in the green (Figure 5a) and blue (Figure 5b) channel, however since the particles are bright, the high signal to noise ratio makes it possible to identify nanoparticles internalized in cells. Figure 5e, shows the overlaid image of the four channels, where cells labeled with hB particles (blue circles) show emission only in the blue channel, with no emission in green or red channel, whereas cells labeled with hGhRmB (magenta circle) show emission in red, green and blue channels. Cells containing dual color particles, hGhB (yellow circle) and hGhR (red circle) show no contribution in the red and blue channels, respectively. Figure 5f shows the bright-field image of the cells showing that the morphology of these RBL-2H3 cells were intact after electroporation, thus making it possible to carry out cell imaging. Based on this figure we show that the *mc*C dots are internalized and from colocalizing the emission contribution from each channel, the nanoparticle stained cells can be distinguished from each other.

This example is the first demonstration of *intra*-cellular fluorescence multiplexing utilizing multicolor silica nanoparticles. The bright *mc*C dots provide a platform to differentiate targets based on a simple RGB color code. It is expected that appropriate nanoparticle surface functionalization of these layer-by-layer particle architectures can provide as a powerful tool towards *in vitro* and *in vivo* applications in fundamental biology, cell signaling, biomedicine and high throughput cellular and pharmaceutical screening.

## EXAMPLE 2

The following is an example describing use of the multilayer, FRM-containing nanoparticles of the present disclosure in imaging methods.

Sample Preparation. In these experiments, each of 26 Rat Basophilic Leukemia (RBL) cell samples were mechanically transfected with a single type of particle via square-wave electroporation. Cells were membrane-labeled using an Alexa Fluor® 488 and cholera toxin conjugate. This fourth spectrally distinct fluorescent dye was chosen so that the fluorescent cell membrane could be distinguished from the *mc* C Dots inside the cells. Single-particle samples were plated directly on glass microscope slides and fixed before imaging. Mixing single-particle cell samples before plating enabled us to create multi-particle samples having only a single type of *mc* C Dot per cell.

*Imaging & Analysis.* Cells were imaged using a Zeiss 710 confocal scanning laser microscope, enabling simultaneous detection of the Cy5, TMR, DAC, and Alexa Fluor® 488 dyes. Images were acquired simultaneously in a "red" channel ($\lambda_{ex}$ = 633 nm), a "green" channel ($\lambda_{ex}$ = 561 nm), a "blue" channel ($\lambda_{ex}$ = 405 nm), and a "yellow" channel ($\lambda_{ex}$

= 488 nm). Individual 16-bit 1280 x 1280 pixel images, four fluorescent images and one bright-field image, were acquired and sectioned into 512 x 512 pixel tiles. A representative tile from a mixed-particle image, showing Alexa Fluor® 488 labeled RBL cells containing up to 17 different particles is shown in Figure 6. The fluorescent images were also stacked in order to show that the cells have taken up a sufficiently high concentration of particles.

Single cells were identified using the fluorescent cell-membrane label. Background fluorescence and cell aggregates were excluded using size analysis, whereby cells were approximated as circular areas of high intensity. Fluorescent entities with diameter below 5 $\mu$m or above 15 $\mu$m were considered to be outside the normal size range of single cells and thus were not analyzed. Single cell boundaries identified in this image were also applied to the corresponding RGB channel images. Only pixel intensities at positions on the cell interior were used in the remainder of this analysis. All image-processing algorithms were home-built and implemented using MATLAB.

Single-particle images (1280 x 1280 pixel) were used to characterize the fluorescent signature of the corresponding *mc* C Dot. Individual cells were assigned mean red, green, and blue intensities, calculated using the brightest 50% of pixels in each cell. Low intensity pixels were excluded from this analysis in order to minimize the contribution from pixels containing few or no particles. Statistical analysis of red, green, and blue mean pixel intensities from more than 1000 cells were used to generate RGB thresholds that enable us to "decode" multi-particle images. Multi-particle images were analyzed cell-by-cell according to the results generated above. We have successfully "decoded" a 17-particle image according to this method as proof of concept. A false-colored version of this image (1280 x 1280 pixels) is shown in Figure 7. In the figure, cells have been color-coded according to the "color" of the nanoparticle inside.

**Claims**

1. A nanoparticle comprising:

    a) a silica core comprising a plurality of a fluorescently responsive material (FRM) covalently bound to the silica network of the core;
    b) 1 to 100 FRM-containing silica layers, each layer comprising a plurality of the FRM covalently bound to the silica network of the FRM-containing silica layer;
    c) one or more FRM-free silica layers, wherein one of the FRM-free silica layers separates the silica core from one of the FRM-containing silica layers and, if present, each adjacent pair of the FRM-containing silica layers is separated by one of the FRM-free silica layers;
    d) an outermost FRM-free silica layer disposed on the outermost FRM-containing silica layer; and
    e) a plurality of poly(ethylene glycol) molecules covalently bound to the outer surface of the outermost FRM-free silica layer;

    wherein each FRM-free silica layer has a thickness of 1 nm to 20 nm.

2. The nanoparticle of claim 1, further comprising one or more moieties covalently bound to the poly(ethylene glycol) molecules covalently bound to the outer surface of the outermost FRM-free silica layer.

3. The nanoparticle of claim 2, wherein the one or more moieties are selected from proteins, peptides, nucleic acids, aptamers, antibodies, antibody fragments, polymers, organic small molecules, and combinations thereof.

4. The nanoparticle of claim 3, wherein the nucleic acids are selected from single-stranded DNA molecules, double-stranded DNA molecules, single-stranded RNA molecules, double-stranded RNA molecules, branched DNA molecules, and combinations thereof.

5. The nanoparticle of claim 1, the nanoparticle having a diameter of 5 nm to 500 nm.

6. The nanoparticle of claim 1, the nanoparticle having a diameter of 5 nm to 100 nm.

7. The nanoparticle of claim 1, wherein each FRM-free silica layer has a thickness such that there is 10% or less measurable energy transfer between the FRM in the core and in an adjacent FRM-containing silica layer or in adjacent FRM-containing silica layers.

8. The nanoparticle of claim 1, wherein the core and all FRM-containing layers have a different FRM.

9. The nanoparticle of claim 1, wherein the FRM is an organic dye.

**10.** The nanoparticle of claim 1, wherein the FRM is selected from N-(7-dimethylamino-4-methylcoumarin-3-yl) (DAC), tetramethylrhodamine-5-maleimide (TMR), Cy5, or a combination thereof.

**11.** A method of making the nanoparticle of claim 1 comprising the steps of:

a) contacting a silica precursor, a plurality of a single type of FRM conjugate precursor, a solvent, and base such that a silica core having a plurality of FRM conjugated to the silica network of the silica core is formed,

b) contacting the material from step a) with a silica precursor and a solvent such that a FRM-free silica layer is formed on the silica core;

c) contacting the material from b) with a silica precursor, a single type of FRM conjugate precursor, a solvent, and base such that a FRM-containing silica layer is formed;

d) optionally, contacting the material from step c) with a silica precursor and a solvent such that a FRM-free silica layer is formed on the silica core and contacting the resulting material with a silica precursor, a single type of FRM conjugate precursor, a solvent, and base such that a FRM-containing silica layer is formed;

e) optionally, repeating step d) a desired number of times, wherein the contacting is to the material from a previously carried out step d);

f) contacting the material from step c), d), or step e) with a silica precursor and a solvent such that an outermost FRM-free silica layer is formed on the outermost FRM-containing layer; and

g) contacting the material from step f) with functionalized PEG molecules such that a nanoparticle having a plurality of PEG molecules covalently bound to the outer surface of the outermost FRM-free silica layer of the nanoparticle is formed.

**12.** The method of claim 11, wherein the PEG molecules are heterobifunctional PEG molecules.

**13.** The method of claim 11, further comprising the step of isolating the nanoparticle.

**14.** An imaging method comprising the steps of:

a) contacting a cell with a plurality of nanoparticles of claim 1; and

b) obtaining a plurality of images of the sample, each image obtained using a different excitation wavelength and a different emission wavelength, wherein each different excitation wavelength is in the absorption spectrum of a different type of FRM present in the nanoparticle and each different emission wavelength is in the emission spectrum of a different type of FRM present in the nanoparticle.

**15.** The imaging method of claim 14, further comprising the step of combining the plurality of images to provide a single image.

**16.** The imaging method of claim 14, wherein the image is obtained by confocal microscopy.

**17.** The imaging method of claim 14, wherein the cell is present in a subject.

**Patentansprüche**

**1.** Ein Nanopartikel, das Folgendes beinhaltet:

a) einen Siliziumdioxidkern, der eine Vielzahl von einem fluoreszierend ansprechenden Material (FRM), das kovalent an das Siliziumdioxidnetzwerk des Kerns gebunden ist, beinhaltet;

b) 1 bis 100 FRM-haltige Siliziumdioxidschichten, wobei jede Schicht eine Vielzahl von dem FRM, das kovalent an das Siliziumdioxidnetzwerk der FRM-haltigen Siliziumdioxidschicht gebunden ist, beinhaltet;

c) eine oder mehrere FRM-freie Siliziumdioxidschichten, wobei eine der FRM-freien Siliziumdioxidschichten den Siliziumdioxidkern von einer der FRM-haltigen Siliziumdioxidschichten trennt, und, falls vorhanden, jedes angrenzende Paar der FRM-haltigen Siliziumdioxidschichten durch eine der FRM-freien Siliziumdioxidschichten getrennt ist;

d) eine äußerste FRM-freie Siliziumdioxidschicht, die auf der äußersten FRM-haltigen Siliziumdioxidschicht angeordnet ist; und

e) eine Vielzahl von Poly(ethylenglycol)-Molekülen, die kovalent an die äußere Oberfläche der äußersten FRM-freien Siliziumdioxidschicht gebunden sind;

wobei jede FRM-freie Siliziumdioxidschicht eine Dicke von 1 nm bis 20 nm aufweist.

2. Nanopartikel nach Anspruch 1, das ferner eine oder mehrere Anteile beinhaltet, die kovalent an die Poly(ethylenglycol)-Moleküle gebunden sind, die kovalent an die äußere Oberfläche der äußersten FRM-freien Siliziumdioxidschicht gebunden sind.

3. Nanopartikel nach Anspruch 2, wobei der eine oder die mehreren Anteile aus Proteinen, Peptiden, Nukleinsäuren, Aptameren, Antikörpern, Antikörperfragmenten, Polymeren, organischen kleinen Molekülen und Kombinationen davon ausgewählt sind.

4. Nanopartikel nach Anspruch 3, wobei die Nukleinsäuren aus einzelsträngigen DNA-Molekülen, doppelsträngigen DNA-Molekülen, einzelsträngigen RNA-Molekülen, doppelsträngigen RNA-Molekülen, verzweigten DNA-Molekülen und Kombinationen davon ausgewählt sind.

5. Nanopartikel nach Anspruch 1, wobei das Nanopartikel einen Durchmesser von 5 nm bis 500 nm aufweist.

6. Nanopartikel nach Anspruch 1, wobei das Nanopartikel einen Durchmesser von 5 nm bis 100 nm aufweist.

7. Nanopartikel nach Anspruch 1, wobei jede FRM-freie Siliziumdioxidschicht eine solche Dicke aufweist, dass eine 10%ige oder geringere messbare Energieübertragung zwischen dem FRM in dem Kern und in einer angrenzenden FRM-haltigen Siliziumdioxidschicht oder in angrenzenden FRM-haltigen Siliziumdioxidschichten stattfindet.

8. Nanopartikel nach Anspruch 1, wobei der Kern und alle FRM-haltigen Schichten ein verschiedenes FRM aufweisen.

9. Nanopartikel nach Anspruch 1, wobei das FRM ein organischer Farbstoff ist.

10. Nanopartikel nach Anspruch 1, wobei das FRM aus N-(7-Dimethylamino-4-methylcumarin-3-yl) (DAC), Tetramethylrhodamin-5-maleimid (TMR), Cy5 oder einer Kombination davon ausgewählt ist.

11. Ein Verfahren zur Herstellung des Nanopartikels nach Anspruch 1, das die folgenden Schritte beinhaltet:

   a) In-Kontakt-Bringen eines Siliziumdioxidvorläufers, einer Vielzahl eines einzigen Typs eines FRM-Konjugatvorläufers, eines Lösungsmittels und einer Base, sodass ein Siliziumdioxidkern mit einer Vielzahl von an das Siliziumdioxidnetzwerk des Siliziumdioxidkerns konjugiertem FRM gebildet wird,
   b) In-Kontakt-Bringen des Materials aus Schritt a) mit einem Siliziumdioxidvorläufer und einem Lösungsmittel, sodass eine FRM-freie Siliziumdioxidschicht auf dem Siliziumdioxidkern gebildet wird;
   c) In-Kontakt-Bringen des Materials aus b) mit einem Siliziumdioxidvorläufer, einem einzelnen Typ von FRM-Konjugatvorläufer, einem Lösungsmittel und einer Base, sodass eine FRM-haltige Siliziumdioxidschicht gebildet wird;
   d) optional In-Kontakt-Bringen des Materials aus Schritt c) mit einem Siliziumdioxidvorläufer und einem Lösungsmittel, sodass eine FRM-freie Siliziumdioxidschicht auf dem Siliziumdioxidkern gebildet wird, und In-Kontakt-Bringen des resultierenden Materials mit einem Siliziumdioxidvorläufer, einem einzelnen Typ von FRM-Konjugatvorläufer, einem Lösungsmittel und einer Base, sodass eine FRM-haltige Siliziumdioxidschicht gebildet wird;
   e) optional gewünschtes mehrmaliges Wiederholen von Schritt d), wobei das In-Kontakt-Bringen mit dem Material aus einem zuvor ausgeführten Schritt d) erfolgt;
   f) In-Kontakt-Bringen des Materials aus Schritt c), d), oder Schritt e) mit einem Siliziumdioxidvorläufer und einem Lösungsmittel, sodass eine äußerste FRM-freie Siliziumdioxidschicht auf der äußersten FRM-haltigen Schicht gebildet wird; und
   g) In-Kontakt-Bringen des Materials aus Schritt f) mit funktionalisierten PEG-Molekülen, sodass ein Nanopartikel mit einer Vielzahl von PEG-Molekülen, die kovalent an die äußere Oberfläche der äußersten FRM-freien Siliziumdioxidschicht des Nanopartikels gebunden sind, gebildet wird.

12. Verfahren nach Anspruch 11, wobei die PEG-Moleküle heterobifunktionelle PEG-Moleküle sind.

13. Verfahren nach Anspruch 11, das ferner den Schritt des Isolierens des Nanopartikels beinhaltet.

14. Ein Bildgebungsverfahren, das die folgenden Schritte beinhaltet:

a) In-Kontakt-Bringen einer Zelle mit einer Vielzahl von Nanopartikeln nach Anspruch 1; und

b) Erhalten einer Vielzahl von Bildern der Probe, wobei jedes Bild unter Verwendung einer verschiedenen Anregungswellenlänge und einer verschiedenen Emissionswellenlänge erhalten wird, wobei jede verschiedene Anregungswellenlänge in dem Absorptionsspektrum eines verschiedenen Typs von in dem Nanopartikel vorliegendem FRM liegt und jede verschiedene Emissionswellenlänge in dem Emissionsspektrum eines verschiedenen Typs von in dem Nanopartikel vorliegendem FRM liegt.

**15.** Bildgebungsverfahren nach Anspruch 14, das ferner den Schritt des Kombinierens der Vielzahl von Bildern beinhaltet, um ein einzelnes Bild bereitzustellen.

**16.** Bildgebungsverfahren nach Anspruch 14, wobei das Bild durch konfokale Mikroskopie erhalten wird.

**17.** Bildgebungsverfahren nach Anspruch 14, wobei die Zelle in einem Individuum vorliegt.

## Revendications

**1.** Nanoparticule comprenant :

a) un noyau de silice comprenant une pluralité d'un matériau réagissant par fluorescence (MRF) lié de façon covalente au réseau de silice du noyau ;
b) de 1 à 100 couches de silice contenant un MRF, chaque couche comprenant une pluralité du MRF lié de façon covalente au réseau de silice de la couche de silice contenant un MRF ;
c) une ou plusieurs couches de silice exemptes de MRF, l'une des couches de silice exemptes de MRF séparant le noyau de silice de l'une des couches de silice contenant un MRF et, le cas échéant, chaque paire adjacente des couches de silice contenant un MRF étant séparée par l'une des couches de silice exemptes de MRF ;
d) une couche de silice exempte de MRF située tout à l'extérieur, disposée sur la couche de silice contenant un MRF située tout à l'extérieur ; et
e) une pluralité de molécules de poly(éthylène glycol) liées de façon covalente à la surface extérieure de la couche de silice exempte de MRF située tout à l'extérieur ;

dans laquelle chaque couche de silice exempte de MRF a une épaisseur de 1 nm à 20 nm.

**2.** Nanoparticule selon la revendication 1, comprenant en outre une ou plusieurs fractions liées de façon covalente aux molécules de poly(éthylène glycol) liées de façon covalente à la surface extérieure de la couche de silice exempte de MRF située tout à l'extérieur.

**3.** Nanoparticule selon la revendication 2, dans laquelle lesdites une ou plusieurs fractions sont sélectionnées parmi des protéines, des peptides, des acides nucléiques, des aptamères, des anticorps, des fragments d'anticorps, des polymères, des petites molécules organiques, et des combinaisons de ceux-ci.

**4.** Nanoparticule selon la revendication 3, dans laquelle les acides nucléiques sont sélectionnés parmi des molécules d'ADN monobrin, des molécules d'ADN à double brin, des molécules d'ARN monobrin, des molécules d'ARN à double brin, des molécules d'ADN ramifiées, et des combinaisons de celles-ci.

**5.** Nanoparticule selon la revendication 1, la nanoparticule ayant un diamètre de 5 nm à 500 nm.

**6.** Nanoparticule selon la revendication 1, la nanoparticule ayant un diamètre de 5 nm à 100 nm.

**7.** Nanoparticule selon la revendication 1, dans laquelle chaque couche de silice exempte de MRF a une épaisseur telle qu'un transfert d'énergie mesurable de 10 % ou moins a lieu entre le MRF dans le noyau et dans une couche de silice adjacente contenant un MRF ou dans des couches de silice adjacentes contenant un MRF.

**8.** Nanoparticule selon la revendication 1, dans laquelle le noyau et toutes les couches contenant un MRF ont un différent MRF.

**9.** Nanoparticule selon la revendication 1, dans laquelle le MRF est un colorant organique.

**10.** Nanoparticule selon la revendication 1, dans laquelle le MRF est sélectionné parmi le N-(7-diméthylamino-4-méthylcoumarine-3-yle) (DAC), le tétraméthylrhodamine-5-maléimide (TMR), le Cy5, ou une combinaison de ceux-ci.

**11.** Procédé de fabrication de la nanoparticule selon la revendication 1, comprenant les étapes qui consistent à :

a) mettre en contact un précurseur de silice, une pluralité d'un seul type de précurseur de conjugué de MRF, un solvant, et une base, de façon à former un noyau de silice comportant une pluralité de MRF conjugué au réseau de silice du noyau de silice,

b) mettre en contact le matériau issu de l'étape a) avec un précurseur de silice et un solvant de façon à former une couche de silice exempte de MRF sur le noyau de silice ;

c) mettre en contact le matériau issu de l'étape b) avec un précurseur de silice, un seul type de précurseur de conjugué de MRF, un solvant, et une base, de façon à former une couche de silice contenant un MRF ;

d) optionnellement, mettre en contact le matériau issu de l'étape c) avec un précurseur de silice et un solvant de façon à former une couche de silice exempte de MRF sur le noyau de silice, et mettre en contact le matériau obtenu avec un précurseur de silice, un seul type de précurseur de conjugué de MRF, un solvant, et une base, de façon à former une couche de silice contenant un MRF ;

e) optionnellement, répéter l'étape d) autant de fois qu'on le souhaite, la mise en contact ayant lieu avec le matériau issu d'une étape d) exécutée précédemment ;

f) mettre en contact le matériau issu de l'étape c), d), ou e) avec un précurseur de silice et un solvant de façon à former une couche de silice exempte de MRF située tout à l'extérieur sur la couche contenant un MRF située tout à l'extérieur ; et

g) mettre en contact le matériau issu de l'étape f) avec des molécules de PEG fonctionnalisé de façon à former une nanoparticule comportant une pluralité de molécules de PEG liées de façon covalente à la surface extérieure de la couche de silice exempte de MRF située tout à l'extérieur de la nanoparticule.

**12.** Procédé selon la revendication 11, dans lequel les molécules de PEG sont des molécules de PEG hétérobifonctionnel.

**13.** Procédé selon la revendication 11, comprenant en outre l'étape d'isolation de la nanoparticule.

**14.** Procédé d'imagerie comprenant les étapes qui consistent à :

a) mettre en contact une cellule avec une pluralité de nanoparticules selon la revendication 1 ; et

b) obtenir une pluralité d'images de l'échantillon, chaque image étant obtenue en utilisant une différente longueur d'onde d'excitation et une différente longueur d'onde d'émission, chaque différente longueur d'onde d'excitation se trouvant dans le spectre d'absorption d'un différent type de MRF présent dans la nanoparticule, et chaque différente longueur d'onde d'émission se trouvant dans le spectre d'émission d'un différent type de MRF présent dans la nanoparticule.

**15.** Procédé d'imagerie selon la revendication 14, comprenant en outre l'étape de combinaison de la pluralité d'images pour produire une seule image.

**16.** Procédé d'imagerie selon la revendication 14, dans lequel l'image est obtenue par microscopie confocale.

**17.** Procédé d'imagerie selon la revendication 14, dans lequel la cellule est présente dans un sujet.

Figures 1a-c

**a**

**b**

**1. D:**  **2. D:**  **3. D:**

mG – TMRm (green)core-silica shell
hG – mG + four alternate TMRm (green)dye layer-silica shell

PEG - silane

PEG - silane

**c** 1

mG  hG

green

**Figures 2a-c**

**mR** – Cy5m (red) core-silica shell
**hG** – **mR** + three alternate Cy5m (red) dye layer-silica shell

red

**mB** – DACm core-silica shell
**hB** – **mB** + four alternate DACm (blue) dye layer-silica shell

blue

green, red

**Figures 2d-f**

green, blue

red, blue

green, red, blue

**Figures 2g-i**

green

red

blue

**Figures 3a-c**

**d**

green

**e**

red

**f**

blue

**Figures 3d-f**

**g**

green

**h**

red

**i**

blue

**Figures 3g-i**

**Figures 4a-b**

Figure 4c

a. Green Ch: 560 nm
b. Red Ch: 633 nm
c. Blue Ch: 405 nm
d. Yellow Ch: 488 nm
e. Overlay
f. Bright Field  50 µm

hB
hGhRmB
hGhB
hGhR

**Figures 5a-f**

**Figures 6a-f**

Figures 7a-b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61767066 A
- US 5830912 A
- US 4774339 A
- US 5187288 A
- US 5248782 A
- US 5274113 A
- US 5433896 A
- US 4810636 A
- US 4812409 A